# EUROPEAN PATENT SPECIFICATION

(11) **EP 4 034 122 B1**
(45) Date of publication and mention of the grant of the patent: **13.08.2025**
(21) Application number: 20789788.5
(22) Date of filing: 25.09.2020
(51) Int. Cl.: A61K 31/519, A61K 9/00, A61P 3/00

(54) **METHODS FOR TREATING HYPERPHENYLALANINEMIA**
VERFAHREN ZUR BEHANDLUNG VON HYPERPHENYLALANINÄMIE
PROCÉDÉS DE TRAITEMENT DE L'HYPERPHÉNYLALANINÉMIE

(30) Priority: 25.09.2019 US 201962905720 P
(43) Date of publication of application: 03.08.2022
(73) Proprietor: PTC Therapeutics MP, Inc., Warren, NJ 07059 (US)
(72) Inventor: SMITH, Niel, South Plainfield, NJ 07080 (US)
(74) Representative: Mewburn Ellis LLP
(86) International application number: PCT/US2020/052871
(87) International publication number: WO 2021/062264

(56) References cited:
- WO-A1-2019/046849
- CURTIUS H CH ET AL: "Atypical phenylketonuria due to tetrahydrobiopterin deficiency. Diagnosis and treatment with tetrahydrobiopterin, dihydrobiopterin and sepiapterin", CLINICA CHIMICA ACTA, ELSEVIER BV, AMSTERDAM, NL, vol. 93, no. 2, 16 April 1979 (1979-04-16), pages 251 - 262, XP024785714, ISSN: 0009-8981, [retrieved on 19790416], DOI: 10.1016/0009-8981(79)90097-4
- SMITH NEIL ET AL: "Phase I clinical evaluation of CNSA-001 (sepiapterin), a novel pharmacological treatment for phenylketonuria and tetrahydrobiopterin deficiencies, in healthy volunteers", MOLECULAR GENETICS AND METABOLISM, vol. 126, no. 4, 10 February 2019 (2019-02-10), pages 406 - 412, XP085697597, ISSN: 1096-7192, DOI: 10.1016/J.YMGME.2019.02.001

## Description

### Technical Field

The present invention relates to the use of sepiapterin in therapy, and more specifically, to sepiapterin, or a pharmaceutically acceptable salt thereof, for use in a method of treating phenylketonuria in a subject having a blood phenylalanine concentration greater than 120 micromole per liter, optionally wherein the subject has a blood phenylalanine concentration greater than 600 micromole per liter; wherein: the subject failed to respond to treatment with sapropterin, or a pharmaceutically acceptable salt thereof, wherein the blood plasma phenylalanine concentration of the subject decreased less than 20% upon administration of sapropterin, or a pharmaceutically acceptable salt thereof; and the method comprises administering to the subject an effective amount of the sepiapterin or pharmaceutically acceptable salt thereof.

### Background of the Invention

Phenylketonuria (PKU) is an inborn error of metabolism caused predominantly by mutations in the phenylalanine hydroxylase (PAH) gene. Gene mutations of PAH result in decreased catalytic activity leading to hyperphenylalaninemia (HPA). There are many different mutations in the PAH gene (>400), resulting in phenotypic variation in the amount of enzyme produced and/or enzyme activity. Subjects with severe forms of PKU have a complete absence or profound deficiency of PAH enzyme activity and typically have very high blood phenylalanine (Phe) levels (>1200 micromole per liter). A partial deficiency of PAH activity results in a lower degree of blood phenylalanine elevation, e.g., about 360 to 1200 micromoles per liter. High levels of phenylalanine are toxic to the brain. If left untreated, severe and irreversible intellectual disability can occur. With the near universal adoption of newborn screening, PKU is diagnosed at birth. It has been described in all ethnic groups and is estimated to occur in approximately 1 in every 10,000 births.

Currently, there is no cure for PKU. Initial treatment consists of prompt institution of phenylalanine dietary restriction supplemented with specifically designed medical foods. Compliance with restrictive diet can be difficult for older children, adolescents, and adults. Tetrahydrobiopterin (BH4) supplementation has demonstrated clinically meaningful lowering of phenylalanine plasma concentrations in approximately 30% of PKU patients. BH4 is the essential cofactor for PAH in the conversion of Phe to tyrosine (Tyr). The increase in intracellular BH4 as a result of BH4 supplementation has been shown to improve the function of PAH resulting in the reduction of plasma phenylalanine. Kuvan^{®} (sapropterin dihydrochloride), a synthetic formulation of BH4, is an approved medicine in several countries that is used for the treatment of hyperphenylalaninemia (HPA) in tetrahydrobiopterin-responsive adult and pediatric patients with PKU. However, control with dietary phenylalanine restriction and/or BH4 supplementation is suboptimal with the majority of patients maintaining elevated phenylalanine plasma concentrations. Another method of treating PKU, pegvaliase-pqpz, which has been approved only for adult patients, involves administration of a phenylalanine-metabolizing enzyme by daily injection; however, this method suffers from possible complications due to anaphylaxis and other side effects, and many of the patients maintain elevated phenylalanine levels despite treatment. Accordingly, there is a need for further methods of treating PKU.

Curtius et al., Clin. Chim. Acta, 1979, Vol. 93, No. 2, pp. 252-262, describes the identification of atypical PKU due to tetrahydrobiopterin (BH4) deficiency and treatment of these BH4 deficiencies with BH4, BH2 and sepiapterin.

WO 2019/046849 A1 (Censa Pharmaceuticals; 07 March 2019) describes compositions of sepiapterin which are useful in the treatment of tetrahydrobiopterin-related disorders (BH4).

Smith et al., Mol. Gen. Metab., 10 February 2019, Vol. 126, No. 4, pp. 406-412, describes a Phase I trial of the use of sepiapterin in healthy subjects.

### Summary of the Invention

The invention is sepiapterin, or a pharmaceutically acceptable salt thereof, for use in a method of treating phenylketonuria in a subject having a blood phenylalanine concentration greater than 120 micromole per liter, optionally wherein the subject has a blood phenylalanine concentration greater than 600 micromole per liter;
wherein:
the subject failed to respond to treatment with sapropterin, or a pharmaceutically acceptable salt thereof, wherein the blood plasma phenylalanine concentration of the subject decreased less than 20% upon administration of sapropterin, or a pharmaceutically acceptable salt thereof; and
the method comprises administering to the subject an effective amount of the sepiapterin or pharmaceutically acceptable salt thereof.

In one embodiment:
the subject is on a phenylalanine-restricted diet.

In one embodiment:
the subject failed to respond to treatment with pegvaliase-pqpz;
optionally wherein the subject discontinued treatment with pegvaliase-pqpz due to an adverse reaction and/or tolerability.

In one embodiment:
(a) the administering reduces the blood phenylalanine concentration of the subject to less than 120 micromole per liter;
(b) the administering reduces the blood phenylalanine concentration of the subject at least 35% compared to the blood phenylalanine concentration prior to administration of sepiapterin, or a pharmaceutically acceptable salt thereof;
(c) the administering reduces the blood phenylalanine concentration of the subject to less than 360 micromole per liter with a phenylalanine consumption of at least about 1500 mg/day; and/or
(d) the administering produces a BH4 concentration of at least 50 ng/mL in the plasma of the subject within 10 hours of administration of sepiapterin, or a pharmaceutically acceptable salt thereof.

In one embodiment:
the effective amount of sepiapterin, or pharmaceutically acceptable salt thereof, is about 20 mg/kg to about 60 mg/kg per dose;
optionally wherein:
   (a) the effective amount of sepiapterin, or pharmaceutically acceptable salt thereof, is about 20 mg/kg per dose;
   (b) the effective amount of sepiapterin, or pharmaceutically acceptable salt thereof, is about 40 mg/kg per dose; or
   (c) the effective amount of sepiapterin, or pharmaceutically acceptable salt thereof, is about 60 mg/kg per dose.

In one embodiment:
the risk of adverse events is reduced compared to a subject administered at least 10 mg/kg sapropterin, or a pharmaceutically acceptable salt thereof.

In one embodiment:
the effective amount of sepiapterin, or pharmaceutically acceptable salt thereof, is administered once daily.

In one embodiment:
the effective amount of sepiapterin, or pharmaceutically acceptable salt thereof, is administered twice daily;
preferably wherein the effective amount of sepiapterin, or pharmaceutically acceptable salt thereof, is administered in two equal doses.

In one embodiment:
the effective amount of sepiapterin, or a pharmaceutically acceptable salt thereof, is administered with food;
optionally wherein:
   (a) administration to the subject occurs less than 30 minutes prior to consuming food or after consuming food;
      preferably wherein the administration to the subject is substantially at the same time as food;
   (b) the food is high protein and/or high fat food; and/or
   (c) the food is high calorie food.

In one embodiment:
the administering produces an increase in neurocognitive function of the subject.

In one embodiment:
the subject is a child;
optionally wherein the child is less than seven years old.

In one embodiment:
the subject is more than seven years old.

In one embodiment:
the subject has been diagnosed with sepiapterin-responsive phenylketonuria.

In one embodiment:
the sepiapterin or pharmaceutically acceptable salt thereof is formulated as an oral powder for suspension;
optionally wherein the sepiapterin or pharmaceutically acceptable salt thereof is administered as a suspension in a flavored suspending vehicle;
optionally wherein the sepiapterin or pharmaceutically acceptable salt thereof is administered in a pharmaceutical composition with no antioxidant.

In one embodiment:
the subject has a blood phenylalanine concentration greater than 1200 micromole per liter.

### Brief Description

References to the methods of treatment by therapy herein are to be interpreted as references to compounds, pharmaceutical compositions and medicaments for use in those methods.

Described herein are methods of reducing the blood phenylalanine concentration in subjects in need thereof by administering sepiapterin, or a pharmaceutically acceptable salt thereof. The inventors have discovered that sepiapterin is effective at reducing blood phenylalanine concentration. In particular, the inventors have discovered that sepiapterin is effective at reducing blood phenylalanine concentrations to levels similar to those of healthy subjects and/or at reducing blood phenylalanine concentrations in subjects that failed to respond to prior treatment with sapropterin dihydrochloride and/or pegvaliase-pqpz.

Also described herein is a method of reducing the level of phenylalanine (e.g., the blood phenylalanine concentration) in a subject (e.g., a subject with hyperphenylalaninemia, a subject with hyperphenylalaninemia caused by phenylketonuria, a subject with phenylketonuria, a subject with tetrahydrobiopterin-responsive phenylketonuria, or a subject with sepiapterin-responsive phenylketonuria). This method includes administering an effective amount of sepiapterin, or a pharmaceutically acceptable salt thereof.

Also described herein is a method of treating hyperphenylalaninemia (e.g., hyperphenylalaninemia caused by phenylketonuria such as tetrahydrobiopterin-responsive phenylketonuria, or a subject with sepiapterin-responsive phenylketonuria) in a subject in need thereof. This method includes administering an effective amount of sepiapterin, or a pharmaceutically acceptable salt thereof.

Also described herein is a method of treating phenylketonuria (e.g., tetrahydrobiopterin-responsive phenylketonuria, , or a subject with sepiapterin-responsive phenylketonuria, classical phenylketonuria, and/or non-classical phenylketonuria) in a subject in need thereof. This method includes administering an effective amount of sepiapterin, or a pharmaceutically acceptable salt thereof.

In the methods described herein, the subject has a blood phenylalanine concentration (e.g., an uncontrolled phenylalanine blood concentration) of greater than 120 micromole per liter, (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter). In some embodiments of any of the methods described herein, the subject has a blood phenylalanine concentration (e.g., an uncontrolled phenylalanine blood concentration) of between 120 and 360 micromole per liter, between 360 and 600 micromole per liter, between 600 and 1200 micromole per liter, or greater than 1200 micromole per liter.

In some embodiments of any of the methods described herein, the subject has a blood phenylalanine concentration (e.g., an uncontrolled phenylalanine blood concentration) of greater than 400 micromole per liter (e.g., greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) on existing management such as a phenylalanine-restricted diet and/or treatment with sapropterin, or a pharmaceutically acceptable salt thereof (e.g., sapropterin dihydrochloride). In some embodiments, the treatment with sapropterin includes administration of 5 to 20 mg/kg of sapropterin, or a pharmaceutically acceptable salt thereof.

In the methods described herein, the subject failed to respond to treatment with sapropterin, or a pharmaceutically acceptable salt thereof (e.g., sapropterin dihydrochloride). The blood plasma concentration of the subject decreased less than 20% upon administration of sapropterin, or a pharmaceutically acceptable salt thereof, (e.g., sapropterin dihydrochloride). In some embodiments, the blood plasma concentration of the subject decreased less than 15% upon administration of sapropterin, or a pharmaceutically acceptable salt thereof, (e.g., sapropterin dihydrochloride). For example, the blood plasma concentration of the subject decreased less than 20% upon administration of at least about 10 mg/kg (e.g., at least about 15 mg/kg, at least about 20 mg/kg) of sapropterin, or a pharmaceutically acceptable salt thereof, (e.g., sapropterin dihydrochloride) for at least eight days (e.g., at least fourteen days, at least 21 days, at least 28 days, at least 30 days). In some embodiments, the blood plasma concentration of the subject was greater than 120 micromole per liter, (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) after administration of sapropterin, or a pharmaceutically acceptable salt thereof, (e.g., sapropterin dihydrochloride). For example, the blood plasma concentration of the subject was greater than 120 micromole per liter, (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) after administration of at least about 10 mg/kg (e.g., at least about 15 mg/kg, at least about 20 mg/kg) of sapropterin, or a pharmaceutically acceptable salt thereof, (e.g., sapropterin dihydrochloride) for at least eight days (e.g., at least fourteen days, at least 21 days, at least 28 days, at least 30 days).

In some embodiments of any of the methods described herein, the subject failed to respond to treatment with pegvaliase-pqpz. In some embodiments, the blood plasma concentration of the subject decreased less than 20% upon administration of pegvaliase-pqpz. For example, the blood plasma concentration of the subject decreased less than 30%, or less than 20% upon administration of at least about 20 mg once daily (e.g., at least about 30 mg once daily, at least about 40 mg once daily) of pegvaliase-pqpz for at least sixteen weeks (e.g., at least eighteen weeks, at least 20 weeks, at least 22 weeks, at least 24 weeks). In some embodiments, the blood plasma concentration of the subject was greater than 120 micromole per liter (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) after administration of pegvaliase-pqpz. For example, the blood plasma concentration of the subject was greater than 120 micromole per liter (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) after administration of at least about 20 mg (e.g., at least about 30 mg, at least about 40 mg) of pegvaliase-pqpz for at least sixteen weeks (e.g., at least eighteen weeks, at least 20 weeks, at least 22 weeks, at least 24 weeks).

In some embodiments of any of the methods described herein, the subject discontinued treatment with pegvaliase-pqpz due to an adverse reaction (e.g., anaphylaxis, an injection site reaction, arthralgia, a hypersensitivity reaction, headache, a generalized skin reaction lasting at least 14 days, pruritus, nausea, abdominal pain, oropharyngeal pain, vomiting, cough, diarrhea, and/or fatigue) and/or tolerability.

In some embodiments of any of the methods described herein, administering sepiapterin, or a pharmaceutically acceptable salt thereof, reduces the blood phenylalanine concentration of the subject to less than 600 micromole per liter. In some embodiments of any of the methods described herein, administering sepiapterin, or a pharmaceutically acceptable salt thereof, reduces the blood phenylalanine concentration of the subject to less than 360 micromole per liter. In some embodiments of any of the methods described herein, administering sepiapterin, or a pharmaceutically acceptable salt thereof, reduces the blood phenylalanine concentration of the subject to less than 120 micromole per liter. In some embodiments of any of the methods described herein, administering sepiapterin, or a pharmaceutically acceptable salt thereof, reduces the blood phenylalanine concentration of the subject to between 120 and 360 micromole per liter. In some embodiments of any of the methods described herein, administering sepiapterin, or a pharmaceutically acceptable salt thereof, reduces the blood phenylalanine concentration of the subject to between 360 and 600 micromole per liter.

In some embodiments of any of the methods described herein, the effective amount of sepiapterin, or a pharmaceutically acceptable salt thereof, is an amount sufficient to reduce the blood phenylalanine concentration of the subject to less than 600 micromole per liter. In some embodiments of any of the methods described herein, the effective amount of sepiapterin, or a pharmaceutically acceptable salt thereof, is an amount sufficient to reduce the blood phenylalanine concentration of the subject to less than 360 micromole per liter. In some embodiments of any of the methods described herein, the effective amount of sepiapterin, or a pharmaceutically acceptable salt thereof, is an amount sufficient to reduce the blood phenylalanine concentration of the subject to less than 120 micromole per liter. In some embodiments of any of the methods described herein, the effective amount of sepiapterin, or a pharmaceutically acceptable salt thereof, is an amount sufficient to reduce the blood phenylalanine concentration of the subject to between 120 and 360 micromole per liter. In some embodiments of any of the methods described herein, the effective amount of sepiapterin, or a pharmaceutically acceptable salt thereof, is an amount sufficient to reduce the blood phenylalanine concentration of the subject to between 360 and 600 micromole per liter.

In some embodiments of any of the methods described herein, administering sepiapterin, or a pharmaceutically acceptable salt thereof, reduces the blood phenylalanine concentration of the subject by at least 10% (e.g., at least 20%, at least 30%, at least 40%, at least 50%, at least 60%, at least 70%, at least 80%, at least 90% or from about 10% to about 30%, from about 20% to about 40%, from about 30% to about 50%, from about 40% to about 60%, from about 50% to about 70%, from about 60% to about 80%, or from about 70% to about 90%). The reduction may be determined after administration over at least 1 week (e.g., at 2 weeks, 3 weeks, 4 weeks, 5 weeks, 6 weeks).

In some embodiments of any of the methods described herein, the effective amount of sepiapterin, or a pharmaceutically acceptable salt thereof, is an amount sufficient to reduce the blood phenylalanine concentration of the subject by at least 10% (e.g., at least 20%, at least 30%, at least 40%, at least 50%, at least 60%, at least 70%, at least 80%, at least 90%, or from about 10% to about 30%, from about 20% to about 40%, from about 30% to about 50%, from about 40% to about 60%, from about 50% to about 70%, from about 60% to about 80%, or about 70% to about 90%).

In some embodiments of any of the methods described herein, administering sepiapterin, or a pharmaceutically acceptable salt thereof, reduces the blood phenylalanine concentration of the subject to less than 600 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, administering sepiapterin, or a pharmaceutically acceptable salt thereof, reduces the blood phenylalanine concentration of the subject to less than 360 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, administering sepiapterin, or a pharmaceutically acceptable salt thereof, reduces the blood phenylalanine concentration of the subject to less than 120 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, administering sepiapterin, or a pharmaceutically acceptable salt thereof, reduces the blood phenylalanine concentration of the subject to between 120 and 360 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, administering sepiapterin, or a pharmaceutically acceptable salt thereof, reduces the blood phenylalanine concentration of the subject to between 360 and 600 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day).

In some embodiments of any of the methods described herein, administering sepiapterin, or a pharmaceutically acceptable salt thereof, reduces the blood phenylalanine concentration of the subject to less than 600 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the foregoing methods, administering sepiapterin, or any pharmaceutically acceptable salt thereof, reduces the blood phenylalanine concentration of the subject to less than 360 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, administering sepiapterin, or any pharmaceutically acceptable salt thereof, reduces the blood phenylalanine concentration of the subject to less than 120 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, administering sepiapterin, or any pharmaceutically acceptable salt thereof, reduces the blood phenylalanine concentration of the subject to between 120 and 360 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, administering sepiapterin, or any pharmaceutically acceptable salt thereof, reduces the blood phenylalanine concentration of the subject to between 360 and 600 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day).

In some embodiments of any of the methods described herein, administering sepiapterin, or a pharmaceutically acceptable salt thereof, produces a reduction of the blood phenylalanine concentration of the subject of at least 20% (e.g., at least 25%, at least 30%, at least 35%, at least 40%, at least 45%, at least 50%, at least 60%, at least 70%, at least 80%, at least 90%, at least 95%) from the blood phenylalanine concentration prior to administration of sepiapterin, or a pharmaceutically acceptable salt thereof.

In some embodiments of any of the methods described herein, administering sepiapterin, or a pharmaceutically acceptable salt thereof, produces a BH4 concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration.

In some embodiments of any of the methods described herein, the effective amount of sepiapterin, or a pharmaceutically acceptable salt thereof, is an amount sufficient to reduce the blood phenylalanine concentration of the subject to less than 600 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, the effective amount of sepiapterin, or a pharmaceutically acceptable salt thereof, is an amount sufficient to reduce the blood phenylalanine concentration of the subject to less than 360 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, the effective amount of sepiapterin, or a pharmaceutically acceptable salt thereof, is an amount sufficient to reduce the blood phenylalanine concentration of the subject to less than 120 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, the effective amount of sepiapterin, or a pharmaceutically acceptable salt thereof, is an amount sufficient to reduce the blood phenylalanine concentration of the subject to between 120 and 360 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments of any of the methods described herein, the effective amount of sepiapterin, or a pharmaceutically acceptable salt thereof, is an amount sufficient to reduce the blood phenylalanine concentration of the subject to between 360 and 600 micromole per liter with a phenylalanine consumption of at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day, or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day).

In some embodiments of any of the methods described herein, the effective amount of sepiapterin, or a pharmaceutically acceptable salt thereof, is an amount sufficient to reduce the blood phenylalanine concentration of the subject to less than 600 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, the effective amount of sepiapterin, or a pharmaceutically acceptable salt thereof, is an amount sufficient to reduce the blood phenylalanine concentration of the subject to less than 360 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, the effective amount of sepiapterin, or a pharmaceutically acceptable salt thereof, is an amount sufficient to reduce the blood phenylalanine concentration of the subject to less than 120 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, the effective amount of sepiapterin, or a pharmaceutically acceptable salt thereof, is an amount sufficient to reduce the blood phenylalanine concentration of the subject to between 120 and 360 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, the effective amount of sepiapterin, or a pharmaceutically acceptable salt thereof, is an amount sufficient to reduce the blood phenylalanine concentration of the subject to between 360 and 600 micromole per liter with a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day).

In some embodiments of any of the methods described herein, the effective amount is an amount sufficient to reduce the blood phenylalanine concentration of the subject at least 20% (e.g., at least 25%, at least 30%, at least 35%, at least 40%, at least 45%, at least 50%, at least 60%, at least 70%, at least 80%, at least 90%, at least 95%) from the blood phenylalanine concentration prior to administration of sepiapterin, or a pharmaceutically acceptable salt thereof.

In some embodiments of any of the methods described herein, the effective amount is an amount (e.g., 2.5 mg/kg to 100 mg/kg per dose) sufficient to produce a BH4 concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration of the sepiapterin or a pharmaceutically acceptable salt thereof..

In some embodiments of any of the methods described herein, the effective amount of sepiapterin, or a pharmaceutically acceptable salt thereof, is about 2.5 mg/kg to 100 mg/kg per dose (e.g., about 20 mg/kg to about 60 mg/kg, or about 20 mg/kg, about 30 mg/kg, about 40 mg/kg, about 50 mg/kg, about 60 mg/kg).

In some embodiments of any of the methods described herein, the effective amount of sepiapterin, or a pharmaceutically acceptable salt thereof, is administered with food. In some embodiments of any of the methods described herein, the effective amount is an amount (e.g., 2.5 mg/kg to 100 mg/kg per dose) sufficient to produce a BH4 concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration with food. In some embodiments, the effective amount includes a dose that is at least 5% (at least 10%, at least 20%, at least 50%, at least 70%, at least 90%, at least 100%, at least 110%, at least 120%, at least 130%, at least 140%, or at least 150%) lower than the dose sufficient to produce a maximum BH4 plasma concentration (Cmax) of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration of sepiapterin, or a pharmaceutically acceptable salt thereof, without food.

In some embodiments of any of the methods described herein, administration to the subject occurs less than 30 minutes prior to consuming food, or after consuming food, e.g., immediately prior to the consumption of food or up to 1 hour after consumption. In some embodiments, the administration to the subject is substantially at the same time as food. In some embodiments of any of the methods described herein, the food is a high protein food. In some embodiments of any of the methods described herein, the food is a high fat food (e.g., at least 25, 30, 40, or 50% of the calories are from fat). In some embodiments of any of the methods described herein, the food is a high protein and high fat food. In some embodiments, the food is high calorie food (e.g., the food includes at least 100 calories, e.g., at least 200 calories, at least 300 calories, at least 400 calories, at least 500 calories, e.g., 500-1500 or 800-1000 calories). In some embodiments of any of the methods described herein, the food is a meal, e.g., breakfast, lunch, or dinner.

In some embodiments of any of the methods described herein, the administration with food (e.g., occurring less than 30 minutes prior to consuming food, or after consuming food, e.g., immediately prior to the consumption of food up to 1 hour after consumption) results in an increase (e.g., at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, or at least 150%) in the Cmax of BH4 compared to administration without food (e.g., occurring more than 2 hours after consuming food until 30 minutes prior to consuming further food).

In some embodiments of any of the methods described herein, the administration with food (e.g., occurring less than 30 minutes prior to consuming food or after consuming food, e.g., immediately prior to the consumption of food up to 1 hour after consumption) results in an increase (e.g., at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, or at least 150%) in the extent of production and resulting plasma exposure (AUC₀₋ₗₐₛₜ) of BH4 compared to administration without food (e.g., occurring less than 30 minutes prior to consuming food or after consuming food, e.g., immediately prior to the consumption of food up to 1 hour after consumption).

In some embodiments of any of the methods described herein, the sepiapterin, or a pharmaceutically acceptable salt thereof, is provided in a separate composition from the consumed food (e.g., the sepiapterin is not incorporated into a food product). In some embodiments of any of the methods described herein, the consumption of food occurs prior to the administration of sepiapterin or a pharmaceutically acceptable salt thereof (e.g., the consumption of food occurs between 1 hour up to immediately prior to the administration of sepiapterin). In some embodiments of any of the methods described herein, the consumption of food occurs after the administration of sepiapterin or a pharmaceutically acceptable salt thereof (e.g., the consumption of food occurs between immediately after administration up to 30 minutes after administration).

In some embodiments of any of the methods described herein, the effective amount of sepiapterin, or a pharmaceutically acceptable salt thereof, is administered in two equal doses (e.g., two doses at different times of day). In some embodiments of any of the methods described herein, the effective amount of sepiapterin, or a pharmaceutically acceptable salt thereof, is administered in two 60 mg/kg doses (e.g., one 60 mg/kg dose in the morning and one 60 mg/kg dose in the evening). In some embodiments of any of the methods described herein, the effective amount of sepiapterin, or a pharmaceutically acceptable salt thereof, is administered in two 40 mg/kg doses (e.g., one 40 mg/kg dose in the morning and one 40 mg/kg dose in the evening). In some embodiments of any of the methods described herein, the effective amount of sepiapterin, or a pharmaceutically acceptable salt thereof, is administered in two 30 mg/kg doses (e.g., one 30 mg/kg dose in the morning and one 30 mg/kg dose in the evening). In some embodiments of any of the methods described herein, the effective amount of sepiapterin, or a pharmaceutically acceptable salt thereof, is administered in two 20 mg/kg doses (e.g., one 20 mg/kg dose in the morning and one 20 mg/kg dose in the evening). In some embodiments of any of the methods described herein, the effective amount of sepiapterin, or a pharmaceutically acceptable salt thereof, is administered in two 10 mg/kg doses (e.g., one 10 mg/kg dose in the morning and one 10 mg/kg dose in the evening).

In some embodiments of any of the methods described herein, the risk of adverse events (e.g., headache, rhinorrhea, pharyngolaryngeal pain, diarrhea, vomiting, cough, and/or nasal congestion) is reduced compared to a subject administered at least 10 mg/kg (e.g., at least 15 mg/kg, at least 20 mg/kg) sapropterin, or a pharmaceutically acceptable salt thereof (e.g., sapropterin dihydrochloride). In some embodiments of any of the methods described herein, the risk of adverse events (e.g., headache, rhinorrhea, pharyngolaryngeal pain, diarrhea, vomiting, cough, and/or nasal congestion) is reduced compared to a subject administered at least 20 mg/kg sapropterin, or a pharmaceutically acceptable salt thereof (e.g., sapropterin dihydrochloride).

In some embodiments of any of the methods described herein, the subject is a child (e.g. the subject is less than 18 years old, less than 17 years old, less than 16 years old, less than 15 years old, less than 14 years old, less than 13 years old, less than 12 years old, less than 11 years old, less than 10 years old, less than 9 years old, less than 8 years old, less than 7 years old, less than 6 years old, less than 5 years old, less than 4 years old, less than 3 years old, less than 2 years old, less than 1 year old). In some embodiments of any of the methods described herein, the subject is an adult (e.g., the subject is greater than 18 years old).

In some embodiments of any of the methods described herein, the subject has been diagnosed with tetrahydrobiopterin-responsive phenylketonuria. In some embodiments of any of the methods described herein, the subject has been diagnosed with hyperphenylalaninemia due to sepiapterin responsive PKU.

In some embodiments of any of the methods described herein, the subject is on a phenylalanine-restricted diet (e.g., the subject is on a diet including a milk substitute or formula such as Phenyl-Free 2 and measured amounts of fruits, vegetables, bread, pasta, and cereals). In some embodiments of any of the methods described herein, the subject has a median phenylalanine intake of less than 3000 mg/day (e.g., less than 2500 mg/day, less than 2000 mg/day, less than 1500 mg/day, less than 1000 mg/day, less than 500 mg/day).

In some embodiments of any of the methods described herein, the subject is not on a phenylalanine-restricted diet (e.g., the subject has a median phenylalanine intake of greater than 1000 mg/day (e.g., greater than 1500 mg/day, greater than 2000 mg/day, greater than 2500 mg/day, greater than 3000 mg/day).

In some embodiments of any of the methods described herein, less than 25% (e.g., less than 20%, less than 15%, less than 10%, or less than 5%) of the protein in the subject's diet is natural protein. In some embodiments of any of the methods described herein, more than 25% (e.g., more than 30%, more than 40%, more than 50%, more than 60%, more than 70%, more than 80%, more than 90%, more than 95%, more than 99%) of the protein in the subject's diet is natural protein. In some embodiments of any of the methods described herein, the subject has a natural protein intake of less than 10 g/day (e.g., less than 5 g/day or from about 5 g/day to about 10 g/day). In some embodiments of any of the methods described herein, the subject has a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day, or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day).

In some embodiments of any of the methods described herein, the sepiapterin or a pharmaceutically acceptable salt thereof, is formulated as an oral powder for suspension. In some embodiments of any of the methods described herein, the sepiapterin or a pharmaceutically acceptable salt thereof, is administered as a suspension in a flavored suspending vehicle (e.g., MEDISCA^{®}Oral Mix).

In some embodiments of any of the methods described herein, the administering produces an increase in neurocognitive function of the subject (e.g., an increase in executive function, a decrease in anxiety, a decrease in attention-deficit/hyperactivity disorder symptoms, and/or a decrease in instances of brain fog). In some embodiments, the administering produces an increase in executive function, e.g., as determined by an assessment such as Behavioural Assessment of Dysexecutive Syndrome (BADS), Behavior Rating Inventory of Executive Function (BRIEF or Mini-BRIEF), Barkley Deficits in Executive Functioning Scales (BDEFS), Behavioral Dyscontrol Scale (BDS),the ASEBA Child Behavior Checklist (CBLC), Comprehensive Executive Function Inventory (CEFI), CogScreen, Continuous Performance Task (CPT), Controlled Oral Word Association Test (COWAT), d2 Test of Attention, Delis-Kaplan Executive Function System (D-KEFS), Digit Vigilance Test, Figural Fluency Test, Halstead Category Test, Hayling and Brixton tests, lowa gambling task, Kaplan Baycrest Neurocognitive Assessment (KBNA), Kaufman Short Neuropsychological Assessment, Mental Clutter Scale, Paced Auditory Serial Addition Test (PASAT), phenylketonuria - quality of life (PKU-QOL), Rey-Osterrieth Complex Figure, Ruff Figural Fluency Test, Stroop task, Tasks of Executive Control, Test of Variables of Attention (T.O.V.A.), Tower of London Test, Trail-Making Test (TMT) or Trails A & B, Wisconsin Card Sorting Test (WCST), or Symbol Digit Modalities Test. In some embodiments of any of the methods described herein, the administering produces an increase in executive function, e.g., as determined by a Cambridge Neuropsychological Test Automated Battery (CANTAB) assessment, e.g., by measuring reaction time, spatial span, spatial working memory, rapid digital information processing, sustained attention, and/or stop signal task. In some embodiments of any of the methods described herein, the administering produces an improvement in attention and/or mood, e.g., as measured by the ADHD-RS 5 scale (or the Inattention assessment thereof) and/or the Profile Mood States (POMS) scale.

In some embodiments of any of the methods described herein, the effective amount is an amount sufficient to produce an increase in neurocognitive function of the subject (e.g., an increase in executive function, a decrease in anxiety, a decrease in attention-deficit/hyperactivity disorder symptoms, and/or a decrease in instances of brain fog). In some embodiments, the effective amount is an amount sufficient to produce an increase in executive function, e.g., as determined by an assessment such as Behavioural Assessment of Dysexecutive Syndrome (BADS), Behavior Rating Inventory of Executive Function (BRIEF or Mini-BRIEF), Barkley Deficits in Executive Functioning Scales (BDEFS), Behavioral Dyscontrol Scale (BDS), the ASEBA Child Behavior Checklist (CBLC), Comprehensive Executive Function Inventory (CEFI), CogScreen, Continuous Performance Task (CPT), Controlled Oral Word Association Test (COWAT), d2 Test of Attention, Delis-Kaplan Executive Function System (D-KEFS), Digit Vigilance Test, Figural Fluency Test, Halstead Category Test, Hayling and Brixton tests, lowa gambling task, Kaplan Baycrest Neurocognitive Assessment (KBNA), Kaufman Short Neuropsychological Assessment, Mental Clutter Scale, Paced Auditory Serial Addition Test (PASAT), phenylketonuria - quality of life (PKU-QOL), Rey-Osterrieth Complex Figure, Ruff Figural Fluency Test, Stroop task, Tasks of Executive Control, Test of Variables of Attention (T.O.V.A.), Tower of London Test, Trail-Making Test (TMT) or Trails A & B, Wisconsin Card Sorting Test (WCST), or Symbol Digit Modalities Test. In some embodiments, the effective amount is an amount sufficient to produce an increase in executive function, e.g., as determined by a Cambridge Neuropsychological Test Automated Battery (CANTAB) assessment, e.g., by measuring reaction time, spatial span, spatial working memory, rapid digital information processing, sustained attention, and/or stop signal task. In some embodiments of any of the methods described herein, the effective amount is an amount sufficient to produce an improvement in attention and/or mood, e.g., as measured by the ADHD-RS 5 scale (or the Inattention assessment thereof) and/or the Profile Mood States (POMS) scale.

In some embodiments of any of the methods described herein, the administering produces an increase in sleep quality and/or a decrease in symptoms associated with sleep deprivation. In some embodiments of any of the methods described herein, the effective amount is an amount sufficient to produce an increase in sleep quality and/or a decrease in symptoms associated with sleep deprivation.

### Definitions

In this application, unless otherwise clear from context, (i) the term "a" may be understood to mean "at least one"; (ii) the term "or" may be understood to mean "and/or"; (iii) the terms "comprising" and "including" may be understood to encompass itemized components or steps whether presented by themselves or together with one or more additional components or steps; and (iv) the term "approximately" may be understood to permit standard variation as would be understood by those of ordinary skill in the art; and (v) where ranges are provided, endpoints are included.

It is to be understood that the description of compounds, compositions, formulations, and methods of treatment described herein include "comprising", "consisting of", and "consisting essentially of" embodiments. In some embodiments, for all compositions described herein, and all methods using a composition described herein, the compositions can either comprise the listed components or steps, or can "consist essentially of" the listed components or steps. When a composition is described as "consisting essentially of" the listed components, the composition contains the components listed, and may contain other components which do not substantially affect the condition being treated, but do not contain any other components which substantially affect the condition being treated other than those components expressly listed; or, if the composition does contain extra components other than those listed which substantially affect the condition being treated, the composition does not contain a sufficient concentration or amount of the extra components to substantially affect the condition being treated. When a method is described as "consisting essentially of" the listed steps, the method contains the steps listed, and may contain other steps that do not substantially affect the condition being treated, but the method does not contain any other steps which substantially affect the condition being treated other than those steps expressly listed. As a non-limiting specific example, when a composition is described as 'consisting essentially of' a component, the composition may additionally contain any amount of pharmaceutically acceptable carriers, vehicles, or diluents and other such components which do not substantially affect the condition being treated.

Unless otherwise clear from context, all references to sepiapterin contained herein refer to sepiapterin or a pharmaceutically acceptable salt of sepiapterin.

As used herein, the term "about" represents a value that is in the range of ±10% of the value that follows the term "about." Reference to "about" a value or parameter herein includes (and describes) variations that are directed to that value or parameter per se. For example, description referring to "about X" includes description of "X".As used herein, the term "administration" refers to the administration of a composition (e.g., a compound or a preparation that includes a compound as described herein) to a subject or system. Administration to an animal subject (e.g., to a human) may be by any appropriate route. For example, in some embodiments, administration may be bronchial (including by bronchial instillation), buccal, enteral, interdermal, intra-arterial, intradermal, intragastric, intramedullary, intramuscular, intranasal, intraperitoneal, intrathecal, intravenous, intraventricular, mucosal, nasal, oral, rectal, subcutaneous, sublingual, topical, tracheal (including by intratracheal instillation), transdermal, vaginal, and vitreal.

By "determining the level of phenylalanine" is meant the detection of phenylalanine, by methods known in the art either directly or indirectly. "Directly determining" means performing a process (e.g., performing an assay or test on a sample or "analyzing a sample" as that term is defined herein) to obtain the physical entity or value. "Indirectly determining" refers to receiving the physical entity or value from another party or source (e.g., a third-party laboratory that directly acquired the physical entity or value). Methods to measure phenylalanine level generally include, but are not limited to, fluorescent assays in which a by-product of a reaction of phenylalanine reacts with a fluorescent probe.

An "effective amount" of a compound may vary according to factors such as the disease state, age, sex, and weight of the individual, and the ability of the compound to elicit the desired response. A therapeutically effective amount encompasses an amount in which any toxic or detrimental effects of the compound are outweighed by the therapeutically beneficial effects. An effective amount also encompasses an amount sufficient to confer benefit, e.g., clinical benefit.

As used herein, the term "failed to respond to a prior therapy" refers to a subject whose condition remained stable, became worse, or improved by less than the accepted standard for a particular therapy, e.g., as described on the FDA-approved label for the therapy, despite treatment with the therapy.

The term "food," as used herein, refers to solid food with sufficient bulk and fat content that it is not rapidly dissolved and absorbed in the stomach. For example, a meal, such as breakfast, lunch, or dinner. The term "with food," as used herein refers to administration of a composition between about 30 minutes prior to to about two hours after eating, e.g., a meal. The terms "without food," "fasted," or "an empty stomach" refer to the condition of not having consumed solid food for at least about 2 hours until about 30 minutes prior to consuming further solid food.

As used herein, the term "hyperphenylalaninemia" refers to a medical condition wherein a subject has an elevated level of phenylalanine in the blood in comparison to a healthy subject. For example, in some embodiments, a subject is considered to have hyperphenylalaninemia if the concentration of phenylalanine in their blood is greater than 60 micromole per liter. In some embodiments, a subject is considered to have hyperphenylalaninemia if the concentration of phenylalanine in their blood is greater than 120 micromole per liter (e.g., from 120 micromole per liter to 2400 micromole per liter).

By "level" is meant a level of phenylalanine, as compared to a reference. The reference can be any useful reference, as defined herein, e.g., the level in the subject prior to administration, i.e., a baseline level. By a "decreased level" or an "increased level" of phenylalanine is meant a decrease or increase in phenylalanine level, as compared to a reference (e.g., a decrease or an increase by about 5%, about 10%, about 15%, about 20%, about 25%, about 30%, about 35%, about 40%, about 45%, about 50%, about 55%, about 60%, about 65%, about 70%, about 75%, about 80%, about 85%, about 90%, about 95%, about 100%, about 150%, about 200%, about 300%, about 400%, about 500%, or more; a decrease or an increase of more than about 10%, about 15%, about 20%, about 50%, about 75%, about 100%, or about 200%, as compared to a reference; a decrease or an increase by less than about 0.01-fold, about 0.02-fold, about 0.1-fold, about 0.3-fold, about 0.5-fold, about 0.8-fold, or less; or an increase by more than about 1.2-fold, about 1.4-fold, about 1.5-fold, about 1.8-fold, about 2.0-fold, about 3.0-fold, about 3.5-fold, about 4.5-fold, about 5.0-fold, about 10-fold, about 15-fold, about 20-fold, about 30-fold, about 40-fold, about 50-fold, about 100-fold, about 1000-fold, or more). A level of phenylalanine may be expressed in mass/vol (e.g., g/dL, mg/mL, µg/mL, ng/mL), concentration/vol (e.g., nanomole per liter, micromole per liter, micromole per milliliter), or percentage relative to total amino acids in a sample.

By "natural protein" is meant protein from a natural source (e.g., animal, plant, or fungus) that includes Phe.

The term "pharmaceutical composition," as used herein, represents a composition containing a compound described herein formulated with a pharmaceutically acceptable excipient. Pharmaceutical compositions can be formulated, for example, for oral administration in unit dosage form (e.g., a tablet, capsule, caplet, gel cap, suspension, solution, or syrup); for topical administration (e.g., as a cream, gel, lotion, or ointment); for intravenous administration (e.g., as a sterile solution free of particulate emboli and in a solvent system suitable for intravenous use); or in any other pharmaceutically acceptable formulation.

As used herein, the term "pharmaceutically acceptable salt" means any salt that within the scope of sound medical judgment is suitable for use in contact with the tissues of humans and animals without undue toxicity, irritation, allergic response and are commensurate with a reasonable benefit/risk ratio. Pharmaceutically acceptable salts are well known in the art. For example, pharmaceutically acceptable salts are described in: Berge et al., J. Pharmaceutical Sciences 66:1-19, 1977 and in Pharmaceutical Salts: Properties, Selection, and Use, (Eds. P.H. Stahl and C.G. Wermuth), Wiley-VCH, 2008. The salts can be prepared in situ during the final isolation and purification of the compounds described herein or separately by reacting a free base group with a suitable organic acid.

Frequently, the compounds are prepared or used as pharmaceutically acceptable salts prepared as addition products of pharmaceutically acceptable acids. Suitable pharmaceutically acceptable acids and methods for preparation of the appropriate salts are well-known in the art. Salts may be prepared from pharmaceutically acceptable non-toxic acids including inorganic and organic acids.

Representative acid addition salts include acetate, adipate, alginate, ascorbate, aspartate, benzenesulfonate, benzoate, besylate, bisulfate, borate, butyrate, camphorate, camphorsulfonate, citrate, cyclopentanepropionate, digluconate, dodecylsulfate, ethanesulfonate, fumarate, gentisate, glucoheptonate, glycerophosphate, glycolate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, and valerate salts.

By "reducing the blood phenylalanine concentration" is meant decreasing the level of phenylalanine in the blood and/or plasma of a subject. The level of phenylalanine may be measured using any method known in the art, e.g., the concentration of phenylalanine in the subject's blood may be measured by dry blood spot or plasma analysis.

By "sepiapterin-responsive phenylketonuria" is meant phenylketonuria in which a subject exhibits a decrease of blood phenylalanine from baseline after treatment with sepiapterin, or a pharmaceutically acceptable salt thereof, for up to one month.

As used herein, the term "substantially free" refers to the qualitative condition of exhibiting total or near-total extent or degree of the absence of a compound or type of compound of interest. One of ordinary skill in the biological arts will understand that biological and chemical phenomena rarely, if ever, can be determined to be zero without doubt, e.g., due to inherent error in any measurement. The term "substantially free" is therefore used herein to capture the potential lack of completeness inherent in many biological and chemical measurements.

As used herein, the term "subject" or "patient" refers to any organism to which a compound or composition in accordance with this disclosure may be administered, e.g., for experimental, diagnostic, prophylactic, and/or therapeutic purposes. Typical subjects include any animal (e.g., mammals such as mice, rats, rabbits, non-human primates, and humans). A subject may seek or be in need of treatment, require treatment, be receiving treatment, be receiving treatment in the future, or be a human or animal who is under care by a trained professional for a particular disease or condition.

As used herein, the terms "treat," "treated," or "treating" mean both therapeutic treatment and prophylactic or preventative measures wherein the object is to prevent or slow down (lessen) an undesired physiological condition, disorder, or disease, or obtain beneficial or desired clinical results. Beneficial or desired clinical results include, but are not limited to, alleviation of symptoms; diminishment of the extent of a condition, disorder, or disease; stabilized (i.e., not worsening) state of condition, disorder, or disease; delay in onset or slowing of condition, disorder, or disease progression; amelioration of the condition, disorder, or disease state or remission (whether partial or total), whether detectable or undetectable; an amelioration of at least one measurable physical parameter, not necessarily discernible by the subject; or enhancement or improvement of condition, disorder, or disease. Treatment includes eliciting a clinically significant response without excessive levels of side effects. Treatment also includes prolonging survival as compared to expected survival if not receiving treatment.

Unless otherwise defined, all technical and scientific terms used herein have the same meaning as commonly understood by one of ordinary skill in the art to which this invention belongs. Methods and materials are described herein for use in the present disclosure; other, suitable methods and materials known in the art can also be used. The materials, methods, and examples are illustrative only and not intended to be limiting.

The details of one or more embodiments of the invention are set forth in the detailed description below. Other features, objects, and advantages of the invention will be apparent from the description and from the claims.

### Brief Description of the Drawings

**FIG. 1** is a graph illustrating the mean absolute reduction in plasma phenylalanine concentration after treatment with PTC923 low dose and PTC923.
**FIG.** 2 is a chart illustrating six treatment sequences into which the patients were randomized.
**FIG. 3** is a graph illustrating the mean absolute change in blood phenylalanine concentration from baseline after treatment with sapropterin, PTC923 low dose, and PTC923.
**FIG.** 4 is a graph illustrating the mean absolute change in blood phenylalanine concentration from baseline after 3 days after treatment with sapropterin, PTC923 low dose, and PTC923.
**FIG. 5** is a graph illustrating the absolute change in blood phenylalanine concentration for each subject after treatment with sapropterin and PTC923.
**FIG. 6** is a graph illustrating the mean absolute change in blood phenylalanine concentration for classical PKU subjects after treatment with sapropterin and PTC923.
**FIG.** 7 is a graph illustrating the mean absolute change in blood phenylalanine concentration in the sensitivity analysis population after treatment with sapropterin, PTC923 low dose, and PTC923.
**FIG. 8** is a graph illustrating the percent increase in responders to treatment with PTC923 compared to treatment with sapropterin.

### Detailed Description

Again, references to the methods of treatment by therapy herein are to be interpreted as references to compounds, pharmaceutical compositions and medicaments for use in those methods.

The present inventors have discovered that sepiapterin is surprisingly effective at reducing blood phenylalanine concentration. In particular, the inventors have discovered that sepiapterin is effective at reducing blood phenylalanine concentrations to levels similar to those of healthy subjects and/or is effective in subjects that failed to respond to prior treatment with sapropterin dihydrochloride and/or pegvaliase-pqpz. Accordingly, described herein are methods for the reduction of blood phenylalanine concentration in a subject by administering sepiapterin, or a pharmaceutically acceptable salt thereof.

### Sepiapterin

Sepiapterin passes into the cell and is converted to 7,8-dihydrobiopterin by sepiapterin reductase. 7,8-dihydrobiopterin is then converted to BH4 via reduction by dihydrofolate reductase.

Sepiapterin has the structure:

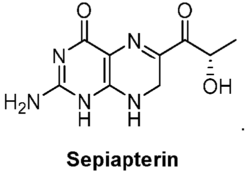

Sepiapterin, or a pharmaceutically acceptable salt thereof, can be formulated in a pharmaceutical composition. In some embodiments, the pharmaceutical composition described herein includes 20-30% sepiapterin, or a salt thereof, by total weight, e.g., about 20%, 22%, 25%, 27%, or 30%. In some embodiments, the pharmaceutical compositions include greater than 20% sepiapterin by total weight, e.g., greater than 25%, greater than 30%, greater than 40%, greater than 50%, greater than 60%, greater than 70%, greater than 80%, or greater than 90%. In some embodiments, the pharmaceutical composition includes less than 20% sepiapterin by total weight, e.g., less than 20%, less than 15%, less than 10%, or less than 5%.

In some embodiments, the pharmaceutical composition may include sepiapterin, or a salt thereof, and less than 10% by total weight of an antioxidant, e.g., about 9%, 7%, 5%, 3%, 1%, 0.5%, 0.25%, 0.1%, or no antioxidant. The antioxidant may be ascorbic acid. In some embodiments, the ratio of sepiapterin, or a pharmaceutically acceptable salt thereof, to the antioxidant is 1:1, or greater than 1:1, e.g., 2:1, 5:1, 7:1, or 10:1 by weight. The pharmaceutical composition may include 20-30% sepiapterin, or a pharmaceutically acceptable salt thereof, by total weight, e.g., about 20%, 22%, 25%, 27%, or 30%. The pharmaceutical composition can further include a dispersant, e.g., croscarmellose sodium. The pharmaceutical composition may include 0.1-1.5% dispersant by total weight, e.g., 0.1%, 0.5%, 1%, or 1.5%. In some embodiments, the pharmaceutical composition includes at least one anti-caking agent, e.g., colloidal silicon dioxide or microcrystalline cellulose. The pharmaceutical composition may include 65-75% anti-caking agent by total weight, e.g., about 65%, 67%, 70%, 73%, or 75%. In some embodiments, the pharmaceutical composition includes both colloidal silicon dioxide and microcrystalline cellulose. In some embodiments, the pharmaceutical composition includes 60-65% microcrystalline cellulose by total weight and 5-7% colloidal silicon dioxide by total weight. In some embodiments, the crystalline form of sepiapterin is formulated as particles less than 140 µm in size, e.g., about 120 µm, 110 µm, 100 µm, 90 µm, 80 µm, 70 µm, 60 µm, 50 µm, 40 µm, 30 µm, 20 µm, 10 µm, or 5 µm. In some embodiments, the pharmaceutical composition includes less than 1% of an impurity by total weight, such as lactoylpterin, e.g., the composition includes less than 0.9%, less than 0.8%, less than 0.7%, less than 0.6%, less than 0.5%, less than 0.4%, less than 0.3%, or less than 0.2%.

In some embodiments, the sepiapterin is a salt of sepiapterin, e.g., with sulfuric acid, p-toluene sulfonic acid, methane sulfonic acid, benzene sulfonic acid, malonic acid, tartaric acid (e.g., L-tartaric acid), phosphoric acid, gentisic acid, fumaric acid, glycolic acid, acetic acid, or nicotinic acid.

In some embodiments, the sepiapterin, or pharmaceutically acceptable salt thereof, is in crystalline form. The crystalline sepiapterin free base or a crystalline form of a salt of sepiapterin can occur as an anhydrate (e.g., without having any bound water or solvent or hydration or solvation) or as a hydrate, a partial hydrate (e.g., hemihydrate, sesquihydrate, and the like), as a dihydrate, a trihydrate, or the like, wherein the crystalline form binds a water of hydration or a solvent molecule associated with the crystalline form of sepiapterin or salt thereof. In an embodiment, crystalline sepiapterin occurs as a monohydrate or as a hemihydrate.

In some embodiments, sepiapterin is present in a crystalline form, e.g., as described in WO 2018/102314 and WO 2018/102315.

In some embodiments, the crystalline form of sepiapterin is characterized by an X-ray powder diffraction pattern obtained by irradiation with Cu Kα X-rays having peaks expressed as 2θ at least at 9.7°±0.5, e.g., 9.7 °±0.2, 10.2°±0.5, e.g., 10.2°±0.2, and 11.3°±0.5, e.g., 11.3°±0.2. In other embodiments, the crystalline form of sepiapterin is characterized by an X-ray powder diffraction pattern obtained by irradiation with Cu Kα X-rays having peaks expressed as 2θ at least at 9.7°±0.5, e.g., 9.7°±0.2, 10.2°±0.5, e.g., 10.2°±0.2, 11.3°±0.5, e.g., 11.3°±0.2, 14.0°±0.5, e.g., 14.0°±0.2, 14.6°±0.5, e.g., 14.6°±0.2, 19.9°±0.5, e.g., 19.9°±0.2, 22.2°±0.5, e.g., 22.2°±0.2, 25.3°±0.5, e.g., 25.3°±0.2, and 32.4°±0.5, e.g., 32.4°±0.2. In an essentially pure form of this crystalline form, peaks can be observed at angles of refraction 2θ as set forth in Table 1. Alternatively or in addition, this crystalline form is characterized by a DSC curve showing two endotherms at 71.6° C and 233.4° C.

**Table 1**

| **Position [2θ°] (±0.5, e.g., ±0.2)** | **Relative Intensity** |
|---|---|
| 9.7 | 98.27 |
| 10.2 | 100.00 |
| 11.3 | 22.47 |
| 14.0 | 5.01 |
| 14.6 | 12.36 |
| 19.9 | 5.63 |
| 21.1 | 3.72 |
| 22.2 | 5.37 |
| 22.7 | 4.04 |
| 24.5 | 2.99 |
| 25.3 | 17.65 |
| 27.2 | 3.10 |
| 32.4 | 5.29 |
| 36.7 | 2.72 |

In some embodiments, the crystalline form of sepiapterin has at least one peak (e.g. one, two, or three peaks) at diffraction angle 2θ (°) of 8.4°±0.5, e.g., 8.4°±0.2, 16.9°±0.5, e.g., 16.9 °±0.2, or 25.4°±0.5, e.g., 25.4°±0.2 as measured by X-ray diffractometry by irradiation with Cu Kα X-rays or calculated from X-ray diffractometry. In some embodiments, the crystalline form of sepiapterin has at least one peak (e.g. one, two, three, four, or five peaks) at diffraction angle 2θ (°) of 8.4°±0.5, e.g., 8.4°±0.2, 14.9°±0.5, e.g.,. 14.9°±0.2, 16.9°±0.5 ,e.g., 16.9°±0.2, 25.4°±0.5, e.g., 25.4°±0.2, and 34.1°±0.5, e.g., 34.1°±0.2 as measured by X-ray diffractometry by irradiation with Cu Kα X-rays or calculated from X-ray diffractometry. In an essentially pure material of this crystalline form, peaks can be observed at angles of refraction 2θ as set forth in Table 2. Alternatively, or in addition, this crystalline form is characterized by a DSC curve showing a melting event at 195.2° C.

**Table 2**

| **Position [2θ°] (±0.5, e.g., ±0.2)** | **Relative Intensity** |
|---|---|
| 8.4 | 100.00 |
| 14.9 | 2.34 |
| 16.9 | 10.70 |
| 25.4 | 84.90 |
| 34.1 | 3.00 |

In some embodiments, the crystalline form of sepiapterin has at least one peak (e.g. one, two, or three peaks) at diffraction angle 2θ (°) of 5.7°±0.5, e.g., 5.7°±0.2, 7.8°±0.5, e.g., 7.8°±0.2, or 25.4°±0.5, e.g., 25.4°±0.2 as measured by X-ray diffractometry by irradiation with Cu Kα X-rays or calculated from X-ray diffractometry. In some embodiments, the crystalline form of sepiapterin has at least one peak (e.g. one, two, three, four, five, six, seven, eight, or nine peaks) at diffraction angle 2θ (°) of 5.7°±0.5, e.g., 5.7°±0.2, 7.8°±0.5, e.g., 7.8°±0.2, 9.1°±0.5, e.g., 9.1°±0.2, 11.5°±0.5, e.g., 11.5°±0.2, 15.3°±0.5, e.g., 15.3°±0.2, 16.0°±0.5, e.g., 16.0°±0.2, 20.1°±0.5, e.g., 20.1°±0.2, 25.4°±0.5, e.g., 25.4°±0.2, and 26.6°±0.5, e.g., 26.6°±0.2, as measured by X-ray diffractometry by irradiation with Cu Kα X-rays or calculated from X-ray diffractometry. In an essentially pure material of this crystalline form, peaks can be observed at angles of refraction 2θ as set forth in Table 3. Alternatively or in addition, this crystalline form is characterized by a DSC curve showing five endothermal peaks at 58.3° C, 101.8° C, 129.8° C, 156.5° C, and 168.3° C.

**Table 3**

| **Position [2θ°] (±0.5, e.g., ±0.2)** | **Relative Intensity** |
|---|---|
| 5.7 | 48.91 |
| 7.8 | 100.00 |
| 9.1 | 59.49 |
| 10.4 | 8.72 |
| 11.5 | 24.53 |
| 12.9 | 8.50 |
| 14.8 | 9.24 |
| 15.3 | 12.53 |
| 16.0 | 14.09 |
| 17.2 | 7.22 |
| 18.2 | 4.25 |
| 19.2 | 5.78 |
| 20.1 | 14.54 |
| 21.5 | 6.47 |
| 22.9 | 6.85 |
| 23.7 | 4.80 |
| 25.4 | 65.68 |
| 26.6 | 14.53 |
| 27.4 | 8.39 |
| 31.5 | 3.74 |
| 34.2 | 4.36 |

In some embodiments, the crystalline form of sepiapterin has at least one peak (e.g. one, two, or three peaks) at diffraction angle 2θ (°) of 8.9°±0.5, e.g., 8.9°±0.2, 10.3°±0.5, e.g., 10.3°±0.2, or 26.0°±0.5, e.g.,26.0°±0.2, as measured by X-ray diffractometry by irradiation with Cu Kα X-rays or calculated from X-ray diffractometry. In some embodiments, the crystalline form of sepiapterin has at least one peak (e.g. one, two, three, four, five, six, seven, eight, or nine peaks) at diffraction angle 2θ (°) of 8.9°±0.5, e.g., 8.9°±0.2, 10.3°±0.5, e.g., 10.3°±0.2, 10.9°±0.5, e.g., 10.9 °±0.2, 17.8°±0.5, e.g., 17.8°±0.2, 24.9°±0.5, e.g., 24.9°±0.2, 26.0°±0.5, e.g., 26.0°±0.2, 26.7°±0.5, e.g., 26.7°±0.2, 26.8°±0.5, e.g., 26.8°±0.2, and 28.3°±0.5, e.g., 28.3°±0.2, as measured by X-ray diffractometry by irradiation with Cu Kα X-rays or calculated from X-ray diffractometry. In an essentially pure material of this crystalline form, peaks can be observed at angles of refraction 2θ as set forth in Table 4. Alternatively or in addition, this crystalline form is characterized by a DSC curve showing three endotherms at 42.7° C, 66.3° C, and 232.9° C.

**Table 4**

| **Position [2θ°] (±0.5, e.g., ±0.2)** | **Relative Intensity** |
|---|---|
| 8.9 | 100.00 |
| 10.3 | 49.92 |
| 10.9 | 19.96 |
| 11.6 | 2.15 |
| 13.6 | 2.99 |
| 14.2 | 3.45 |
| 14.8 | 2.35 |
| 15.4 | 2.59 |
| 16.4 | 1.55 |
| 17.2 | 2.33 |
| 17.8 | 6.24 |
| 19.6 | 2.62 |
| 20.1 | 2.28 |
| 20.5 | 3.09 |
| 20.8 | 2.27 |
| 21.3 | 3.60 |
| 22.3 | 4.79 |
| 23.7 | 4.31 |
| 24.9 | 5.19 |
| 26.0 | 41.94 |
| 26.7 | 8.58 |
| 26.8 | 9.17 |
| 27.4 | 3.98 |
| 28.3 | 4.75 |
| 28.7 | 6.60 |
| 29.8 | 3.03 |
| 31.8 | 2.72 |
| 33.0 | 2.03 |
| 35.5 | 1.57 |
| 37.1 | 1.09 |

In some embodiments, the crystalline form of sepiapterin has at least one peak (e.g. one, two, or three peaks) at diffraction angle 2θ (°) of 4.7°±0.5, e.g., 4.7°±0.2, 7.4°±0.5, e.g., 7.4°±0.2, or 26.2°±0.5, e.g., 26.2°±0.2, as measured by X-ray diffractometry by irradiation with Cu Kα X-rays or calculated from X-ray diffractometry. In some embodiments, the crystalline form of sepiapterin has at least one peak (e.g. one, two, three, four, five, six, seven, or eight peaks) at diffraction angle 2θ (°) of 4.7°±0.5, e.g., 4.7°±0.2, 7.4°±0.5, e.g., 7.4°±0.2, 9.5°±0.5, e.g., 9.5°±0.2, 11.3°±0.5, e.g., 11.3°±0.2, 15.6°±0.5, e.g., 15.6°±0.2, 16.4°±0.5, e.g., 16.4°±0.2, 26.2°±0.5, e.g., 26.2°±0.2, or 27.2°±0.5, e.g., 27.2°±0.2 as measured by X-ray diffractometry by irradiation with Cu Kα X-rays or calculated from X-ray diffractometry. In an essentially pure material of this crystalline form, peaks can be observed at angles of refraction 2θ as set forth in Table 5. Alternatively, or in addition, this crystalline form is characterized by a DSC curve showing endothermal peaks at 82.8° C and 179.8° C.

**Table 5**

| **Position [2θ°] (±0.5, e.g., ±0.2)** | **Relative Intensity** |
|---|---|
| 4.7 | 47.76 |
| 7.4 | 100.00 |
| 9.5 | 33.54 |
| 11.3 | 19.31 |
| 12.4 | 8.49 |
| 13.4 | 3.60 |
| 14.2 | 8.24 |
| 15.6 | 15.08 |
| 16.4 | 11.97 |
| 17.6 | 8.35 |
| 18.4 | 5.03 |
| 19.8 | 9.18 |
| 21.5 | 5.44 |
| 24.4 | 5.56 |
| 26.2 | 35.37 |
| 27.2 | 19.11 |
| 28.9 | 5.93 |

In some embodiments, the crystalline form of sepiapterin has at least one peak (e.g. one, two, three, four, five, or six peaks) at diffraction angle 2θ (°) of 6.0°±0.5, 6.0 °±0.2 10.6°±0.5, 10.6 °±0.2, 12.1°±0.5, e.g., 12.1°±0.2, 15.9°±0.5, e.g., 15.9°±0.2, 20.9°±0.5, e.g., 20.9°±0.2, or 24.6°±0.5, e.g., 24.6°±0.2, as measured by X-ray diffractometry by irradiation with Cu Kα X-rays or calculated from X-ray diffractometry. In some embodiments, the crystalline form of sepiapterin has at least one peak (e.g. one, two, three, four, five, six, seven, eight, nine, ten, eleven, twelve, or thirteen peaks) at diffraction angle 2θ (°) of 6.0°±0.5, e.g., 6.0°±0.2, 10.6°±0.5, e.g., 10.6°±0.2, 12.1°±0.5, e.g., 12.1°±0.2, 15.9°±0.5, e.g., 15.9°±0.2, 18.1°±0.5, e.g., 18.1°±0.2, 20.9°±0.5, e.g., 20.9°±0.2, 22.1°±0.5, e.g., 22.1°±0.2, 24.6°±0.5, e.g., 24.6°±0.2, 26.1°±0.5, e.g., 26.1°±0.2, 28.1°±0.5, e.g., 28.1°±0.2, 28.9°±0.5, e.g., 28.9°±0.2, 32.1°±0.5, e.g., 32.1°±0.2, or 37.0°±0.5, e.g., 37.0°±0.2 as measured by X-ray diffractometry by irradiation with Cu Kα X-rays or calculated from X-ray diffractometry. In an essentially pure form of this crystalline form, peaks can be observed at angles of refraction 2θ as set forth in Table 6. Alternatively or in addition, this crystalline form is characterized by a DSC curve showing two endothermal peaks at 112.9° C and 195.8° C.

**Table 6**

| **Position [2θ°] (±0.5, e.g., ±0.2)** | **Relative Intensity** |
|---|---|
| 6.0 | 100.00 |
| 10.6 | 20.78 |
| 12.1 | 31.95 |
| 15.9 | 12.83 |
| 18.1 | 3.39 |
| 20.9 | 11.63 |
| 22.1 | 2.79 |
| 24.6 | 8.28 |
| 26.1 | 0.88 |
| 28.1 | 7.33 |
| 28.9 | 3.77 |
| 32.1 | 3.57 |
| 37.0 | 1.03 |

In some embodiments, the crystalline form of sepiapterin has at least one peak (e.g. one, two, or three peaks) at diffraction angle 2θ (°) of 10.0°±0.5, e.g., 10.0°±0.2, 10.6°±0.5, e.g., 10.6°±0.2, or 25.7°±0.5, e.g., 25.7°±0.2, as measured by X-ray diffractometry by irradiation with Cu Kα X-rays or calculated from X-ray diffractometry. In some embodiments, the crystalline form of sepiapterin has at least one peak (e.g. one, two, three, four, five, six, seven, eight, nine, or ten peaks) at diffraction angle 2θ (°) of 10.0°±0.5, e.g., 10.0°±0.2, 10.6°±0.5, e.g., 10.6°±0.2, 11.2°±0.5, e.g., 11.2°±0.2, 15.3°±0.5, e.g., 15.3°±0.2, 15.9°±0.5, e.g., 15.9°±0.2, 22.8°±0.5, e.g., 22.8°±0.2, 24.4°±0.5, e.g., 24.4°±0.2, 25.0°±0.5, e.g., 25.0°±0.2, 25.7°±0.5, e.g., 25.7°±0.2, or 26.6°±0.5, e.g., 26.6°±0.2 as measured by X-ray diffractometry by irradiation with Cu Kα X-rays or calculated from X-ray diffractometry. In an essentially pure material of this crystalline form, peaks can be observed at angles of refraction 2θ as set forth in Table 7.

**Table 7**

| **Position [2θ°] (±0.5, e.g., ±0.2)** | **Relative Intensity** |
|---|---|
| 5.3 | 8.30 |
| 6.9 | 4.54 |
| 10.0 | 100.00 |
| 10.6 | 69.64 |
| 11.2 | 6.59 |
| 13.5 | 7.52 |
| 15.3 | 26.59 |
| 15.9 | 26.43 |
| 16.0 | 23.41 |
| 16.9 | 4.28 |
| 18.6 | 13.02 |
| 19.3 | 11.90 |
| 20.1 | 7.22 |
| 20.8 | 11.01 |
| 22.8 | 16.77 |
| 23.5 | 19.60 |
| 24.4 | 41.45 |
| 25.0 | 23.99 |
| 25.7 | 65.40 |
| 26.6 | 39.64 |
| 27.6 | 13.04 |
| 28.7 | 6.55 |
| 30.8 | 14.76 |
| 32.2 | 9.63 |
| 33.7 | 5.16 |
| 37.5 | 5.80 |

In some embodiments, the crystalline form of the hydrochloride salt of sepiapterin has at least one peak (e.g., one, two, or three peaks) at diffraction angle 2θ (°) of 7.8°±0.5, e.g., 7.8°±0.2, 12.9°±0.5, e.g., 12.9°±0.2, or 26.2°±0.5, e.g., 26.2°±0.2, as measured by X-ray diffractometry by irradiation with Cu Kα X-rays or calculated from X-ray diffractometry. In some embodiments, the most intense peak in the X-ray diffraction diagram of the crystalline form of the hydrochloride salt of sepiapterin is observed at an angle of refraction 2θ of 7.8°±0.5, e.g., 7.8°±0.2.In an essentially pure material of this crystalline hydrochloride salt of sepiapterin, peaks can be observed at angles of refraction 2θ as set forth in Table 8. Alternatively or in addition, the crystalline hydrochloride salt of sepiapterin is characterized by a DSC curve showing an endotherm at 225.9° C.

**Table 8**

| **Position [2θ°] (±0.5, e.g., ±0.2)** | **Relative Intensity** |
|---|---|
| 7.8 | 100.00 |
| 8.9 | 6.89 |
| 12.9 | 58.56 |
| 15.6 | 8.52 |
| 17.9 | 25.23 |
| 19.2 | 5.48 |
| 21.1 | 10.97 |
| 23.6 | 25.15 |
| 25.2 | 22.66 |
| 26.2 | 45.91 |
| 27.6 | 32.94 |
| 30.3 | 10.50 |
| 31.7 | 7.83 |
| 34.2 | 8.87 |
| 36.7 | 3.67 |

In some embodiments, the crystalline Form 1 methanesulfonate salt of sepiapterin is characterized by peaks in the X-ray diffraction diagram observed at an angle of refraction 2θ at least at 7.8°±0.5, e.g., 7.8°±0.2, 23.5°±0.5, e.g., 23.5°±0.2, and 29.0°±0.5, e.g., 29.0°±0.2. In some embodiments, the most intense peak in the X-ray diffraction diagram is observed at an angle of refraction 2θ of 23.5°±0.5, e.g., 23.5°±0.2. In an essentially pure material of the crystalline Form 1 methanesulfonate salt of sepiapterin, peaks can be observed at angles of refraction 2θ as set forth in Table 9. Alternatively or in addition, the crystalline form 1 methanesulfonate salt of sepiapterin is characterized by a DSC curve showing two endotherms at 186.0° C and 229.1° C

**Table 9**

| **Position [2θ°] (±0.5, e.g., ±0.2)** | **Relative Intensity** |
|---|---|
| 7.9 | 21.77 |
| 11.7 | 8.20 |
| 13.7 | 8.52 |
| 15.7 | 4.79 |
| 16.6 | 5.34 |
| 18.0 | 5.66 |
| 19.8 | 2.10 |
| 20.3 | 5.36 |
| 20.9 | 2.43 |
| 22.3 | 4.25 |
| 22.7 | 2.15 |
| 23.5 | 100.00 |
| 24.7 | 3.69 |
| 25.6 | 2.70 |
| 26.8 | 1.79 |
| 27.2 | 1.68 |
| 28.3 | 2.75 |
| 29.0 | 57.60 |
| 29.8 | 5.18 |
| 30.5 | 1.37 |
| 32.2 | 4.66 |
| 33.0 | 1.64 |
| 36.5 | 1.29 |

In some embodiments, the crystalline Form 2 methanesulfonate salt of sepiapterin is characterized by peaks in the X-ray diffraction diagram observed at an angle of refraction 2θ at least at 7.9°±0.5, e.g., 7.9°±0.2, 23.4°±0.5, e.g., 23.4°±0.2, and 28.9°±0.5, e.g., 28.9°±0.2. In some embodiments, the most intense peak in the X-ray diffraction diagram is observed at an angle of refraction 2θ of 23.5°±0.5, e.g., 23.5°±0.2. In an essentially pure material of the crystalline Form 2 methanesulfonate salt of sepiapterin, peaks can be observed at angles of refraction 2θ as set forth in Table 10. Alternatively or in addition, the crystalline form 2 methanesulfonate salt of sepiapterin is characterized by a DSC curve showing three endotherms at 75.5° C, 182.6° C, and 234.9° C.

**Table 10**

| **Position [2θ°] (±0.5, e.g., ±0.2)** | **Relative Intensity** |
|---|---|
| 7.9 | 100.00 |
| 11.0 | 21.32 |
| 12.1 | 22.02 |
| 13.5 | 79.87 |
| 15.7 | 11.87 |
| 17.8 | 9.81 |
| 19.7 | 10.93 |
| 21.3 | 26.79 |
| 23.4 | 96.13 |
| 24.1 | 24.88 |
| 24.3 | 22.10 |
| 25.5 | 9.45 |
| 26.0 | 11.27 |
| 27.6 | 7.63 |
| 28.9 | 95.64 |
| 31.2 | 4.39 |
| 36.1 | 6.65 |

In some embodiments, the crystalline Form 3 methanesulfonate salt of sepiapterin is characterized by peaks in the X-ray diffraction diagram observed at an angle of refraction 2θ at least at 21.7°±0.5, e.g., 21.7°±0.2, 26.0°±0.5, e.g., 26.0°±0.2, and 28.9°±0.5, e.g., 28.9°±0.2. In some embodiments, the most intense peak in the X-ray diffraction diagram is observed at an angle of refraction 2θ of 26.0°±0.5, e.g., 26.0°±0.2. In an essentially pure material of the crystalline Form 3 methanesulfonate salt of sepiapterin, peaks can be observed at angles of refraction 2θ as set forth in Table 11. Alternatively or in addition, the crystalline form 3 methanesulfonate salt of sepiapterin is characterized by a DSC curve showing two endotherms at 195.1° C and 240.1° C.

**Table 11**

| **Position [2θ°] (±0.5, e.g., ±0.2)** | **Relative Intensity** |
|---|---|
| 8.2 | 47.29 |
| 10.8 | 56.14 |
| 12.6 | 16.34 |
| 13.2 | 15.90 |
| 14.0 | 24.39 |
| 15.0 | 12.03 |
| 15.9 | 16.20 |
| 18.2 | 22.97 |
| 20.1 | 25.53 |
| 20.5 | 14.97 |
| 21.3 | 22.70 |
| 21.7 | 71.48 |
| 22.2 | 11.40 |
| 23.6 | 46.37 |
| 24.8 | 44.00 |
| 25.5 | 9.08 |
| 26.1 | 100.00 |
| 27.3 | 3.52 |
| 28.9 | 68.42 |
| 31.2 | 4.49 |
| 32.1 | 6.48 |
| 34.8 | 5.95 |
| 35.6 | 1.67 |
| 39.1 | 2.91 |

In some embodiments, the crystalline nicotinate salt of sepiapterin is characterized by peaks in the X-ray diffraction diagram observed at an angle of refraction 2θ at least at 9.5°±0.5, e.g., 9.5°±0.2, 9.9°±0.5, e.g., 9.9°±0.2, and 24.5°±0.5, e.g., 24.5°±0.2. In some embodiments, the most intense peak in the X-ray diffraction diagram is observed at an angle of refraction 2θ of 24.5°±0.5, e.g., 24.5°±0.2. In an essentially pure material of the crystalline nicotinate salt of sepiapterin, peaks can be observed at angles of refraction 2θ as set forth in Table 12. Alternatively or in addition, the crystalline nicotinate salt of sepiapterin is characterized by a DSC curve showing an endotherm at 221.9° C.

**Table 12**

| **Position [2θ°] (±0.5, e.g., ±0.2)** | **Relative Intensity** |
|---|---|
| 9.5 | 10.29 |
| 9.9 | 53.95 |
| 11.5 | 9.31 |
| 12.0 | 11.76 |
| 14.7 | 14.20 |
| 15.9 | 17.61 |
| 17.5 | 7.53 |
| 19.0 | 5.37 |
| 20.8 | 5.88 |
| 21.3 | 6.12 |
| 21.7 | 7.20 |
| 23.2 | 34.05 |
| 24.5 | 100.00 |
| 25.2 | 12.90 |
| 28.0 | 8.51 |
| 31.1 | 5.39 |
| 32.3 | 4.52 |
| 33.4 | 8.02 |
| 35.1 | 5.05 |

In some embodiments, the crystalline p-toluenesulfonate salt of sepiapterin is characterized by peaks in the X-ray diffraction diagram observed at an angle of refraction 2θ at least at 6.5°±0.5, e.g., 6.5°±0.2, 15.1°±0.5, e.g., 15.1°±0.2, and 23.4°±0.5, e.g., 23.4°±0.2. In some embodiments, the most intense peak in the X-ray diffraction diagram is observed at an angle of refraction 2θ of 6.5°±0.5, e.g., 6.5°±0.2. In an essentially pure material of the p-toluenesulfonate salt of sepiapterin, peaks can be observed at angles of refraction 2θ as set forth in Table 13. Alternatively or in addition, the crystalline p-toluenesulfonate salt of sepiapterin is characterized by a DSC curve showing three endotherms at 77.2° C, 202.4° C and 260.2° C.

**Table 13**

| **Position [2θ°] (±0.5, e.g., ±0.2)** | **Relative Intensity** |
|---|---|
| 6.5 | 100.00 |
| 12.9 | 1.79 |
| 14.3 | 1.39 |
| 15.1 | 15.36 |
| 16.2 | 5.33 |
| 18.4 | 8.96 |
| 19.6 | 3.06 |
| 20.2 | 4.86 |
| 21.8 | 2.23 |
| 22.5 | 2.95 |
| 23.1 | 7.99 |
| 23.4 | 9.14 |
| 24.5 | 1.81 |
| 26.0 | 2.48 |
| 27.0 | 4.49 |
| 27.3 | 3.93 |
| 28.1 | 5.31 |
| 28.4 | 5.59 |
| 28.8 | 2.05 |
| 30.6 | 2.24 |
| 31.0 | 1.98 |
| 32.6 | 1.82 |

In some embodiments, the crystalline benzenesulfonate salt of sepiapterin is characterized by peaks in the X-ray diffraction diagram observed at an angle of refraction 2θ at least at 6.5°±0.5, e.g., 6.5°±0.2, 14.8°±0.5, e.g., 14.8°±0.2, and 19.6°±0.5, e.g., 19.6°±0.2. In some embodiments, the most intense peak in the X-ray diffraction diagram is observed at an angle of refraction 2θ of 6.5°±0.5, e.g., 6.5°±0.2. In an essentially pure material of the benzenesulfonate salt of sepiapterin, peaks can be observed at angles of refraction 2θ as set forth in Table 14. Alternatively or in addition, the crystalline benzenesulfonate salt of sepiapterin is characterized by a DSC curve showing two endotherms at 202.3° C and 265.5° C.

**Table 14**

| **Position [2θ°] (±0.5, e.g., ±0.2)** | **Relative Intensity** |
|---|---|
| 4.9 | 5.90 |
| 6.5 | 100.00 |
| 14.8 | 16.73 |
| 17.8 | 4.23 |
| 19.6 | 7.98 |
| 21.5 | 2.49 |
| 23.7 | 3.46 |
| 24.5 | 3.84 |
| 26.1 | 3.29 |

In some embodiments, the crystalline phosphate salt of sepiapterin is characterized by peaks in the X-ray diffraction diagram observed at an angle of refraction 2θ at least at 16.6°±0.5, e.g., 16.6°±0.2, 22.2°±0.5, e.g., 22.2°±0.2, and 25.6°±0.5, e.g., 25.6°±0.2. In some embodiments, the most intense peak in the X-ray diffraction diagram is observed at an angle of refraction 2θ of 25.6°±0.5, e.g., 25.6°±0.2. In an essentially pure material of the crystalline phosphate salt of sepiapterin, peaks can be observed at angles of refraction 2θ as set forth in Table 15. Alternatively or in addition, the crystalline phosphate salt of sepiapterin is characterized by a DSC curve showing three endotherms at 125.9° C, 152.1° C, and 157.6° C.

**Table 15**

| **Position [2θ°] (±0.5, e.g., ±0.2)** | **Relative Intensity** |
|---|---|
| 5.5 | 4.41 |
| 8.1 | 1.21 |
| 8.9 | 2.21 |
| 10.3 | 1.79 |
| 10.8 | 5.80 |
| 15.3 | 1.84 |
| 16.6 | 8.35 |
| 17.7 | 1.95 |
| 20.3 | 1.40 |
| 21.2 | 1.61 |
| 22.2 | 9.77 |
| 23.1 | 1.74 |
| 25.6 | 100.00 |
| 30.8 | 6.31 |
| 31.1 | 4.85 |
| 33.5 | 0.73 |
| 36.0 | 1.70 |

In some embodiments, the crystalline malonate salt of sepiapterin is characterized by peaks in the X-ray diffraction diagram observed at an angle of refraction 2θ at least at 6.9°±0.5, e.g., 6.9°±0.2, 22.7°±0.5, e.g., 22.7°±0.2 and 23.8°±0.5, e.g., 23.8°±0.2. In some embodiments, the most intense peak in the X-ray diffraction diagram is observed at an angle of refraction 2θ of 6.9°±0.5, e.g., 6.9°±0.2. In an essentially pure material of the crystalline malonate salt of sepiapterin, peaks can be observed at angles of refraction 2θ as set forth in Table 16. Alternatively or in addition, the crystalline malonate salt of sepiapterin is characterized by a DSC curve showing a melting event at 115.8° C.

**Table 16**

| **Position [2θ°] (±0.5, e.g., ±0.2)** | **Relative Intensity** |
|---|---|
| 6.9 | 100.00 |
| 8.4 | 13.11 |
| 10.6 | 7.62 |
| 16.4 | 5.63 |
| 17.8 | 9.73 |
| 19.3 | 8.96 |
| 20.1 | 9.99 |
| 22.2 | 10.50 |
| 22.7 | 20.52 |
| 23.8 | 34.02 |
| 24.5 | 5.82 |
| 25.5 | 24.50 |
| 26.6 | 4.00 |
| 27.3 | 6.96 |
| 29.8 | 5.38 |
| 33.1 | 12.08 |

In some embodiments, the crystalline L-tartrate salt of sepiapterin is characterized by peaks in the X-ray diffraction diagram observed at an angle of refraction 2θ at least at 7.3°±0.5, e.g., 7.3°±0.2, 14.2°±0.5, e.g., 14.2°±0.2, and 21.8°±0.5, e.g., 21.8°±0.2. In some embodiments, the most intense peak in the X-ray diffraction diagram is observed at an angle of refraction 2θ of 6.9°±0.5, e.g., 6.9°±0.2. In an essentially pure material of the crystalline L-tartrate salt of sepiapterin, peaks can be observed at angles of refraction 2θ as set forth in Table 17. Alternatively or in addition, the crystalline L-tartrate salt of sepiapterin is characterized by a DSC curve showing two endotherms at 97.2° C and 160.6° C.

**Table 17**

| **Position [2θ°] (±0.5, e.g., ±0.2)** | **Relative Intensity** |
|---|---|
| 7.4 | 100.00 |
| 10.1 | 47.99 |
| 14.2 | 82.76 |
| 14.7 | 27.06 |
| 19.1 | 21.16 |
| 20.2 | 29.91 |
| 21.8 | 85.30 |
| 22.1 | 53.68 |
| 23.9 | 85.30 |
| 24.9 | 19.26 |
| 25.5 | 28.45 |
| 26.8 | 18.58 |
| 29.7 | 21.59 |
| 31.6 | 10.10 |
| 32.9 | 22.18 |

In some embodiments, the crystalline gentisate salt of sepiapterin is characterized by peaks in the X-ray diffraction diagram observed at an angle of refraction 2θ at least at 7.1°±0.5, e.g., 7.1°±0.2, 8.7°±0.5, e.g., 8.7°±0.2, and 26.7°±0.5, e.g., 26.7°±0.2. In some embodiments the most intense peak in the X-ray diffraction diagram is observed at an angle of refraction 2θ of 7.1°±0.5, e.g., 7.1°±0.2. In an essentially pure material of the crystalline gentisate salt of sepiapterin, peaks can be observed at angles of refraction 2θ as set forth in Table 18. Alternatively or in addition, the crystalline gentisate salt of sepiapterin is characterized by a DSC curve showing three endotherms at 70.5° C, 128.2° C, and 184.7° C.

**Table 18**

| **Position [2θ°] (±0.5, e.g., ±0.2)** | **Relative Intensity** |
|---|---|
| 5.7 | 17.29 |
| 7.1 | 100.00 |
| 8.7 | 42.69 |
| 10.4 | 3.94 |
| 11.3 | 11.69 |
| 12.1 | 4.13 |
| 14.3 | 21.10 |
| 16.0 | 6.46 |
| 16.4 | 5.94 |
| 17.0 | 5.85 |
| 17.6 | 7.93 |
| 19.1 | 8.27 |
| 20.20 | 3.47 |
| 20.7 | 2.90 |
| 21.5 | 3.37 |
| 23.6 | 2.69 |
| 24.4 | 4.50 |
| 26.7 | 52.20 |
| 27.1 | 35.49 |
| 28.2 | 8.74 |
| 28.9 | 4.31 |
| 29.9 | 2.62 |
| 31.4 | 2.99 |
| 34.4 | 1.28 |
| 35.8 | 3.54 |
| 37.6 | 0.57 |

In some embodiments, the crystalline fumarate salt of sepiapterin is characterized by peaks in the X-ray diffraction diagram observed at an angle of refraction 2θ at least at 11.3°±0.5, e.g., 11.3°±0.2, 24.0°±0.5, e.g., 24.0°±0.2, and 28.2°±0.5, e.g., 28.2°±0.2. In some embodiments, the most intense peak in the X-ray diffraction diagram is observed at an angle of refraction 2θ of at least 24.0°±0.5, e.g., 24.0°±0.2. In an essentially pure material of the crystalline fumarate salt of sepiapterin, peaks can be observed at angles of refraction 2θ as set forth in Table 19. Alternatively or in addition, the crystalline fumarate salt of sepiapterin is characterized by a DSC curve showing two endotherms at 114.3° C and 229.7° C.

**Table 19**

| **Position [2θ°] (±0.5, e.g., ±0.2)** | **Relative Intensity** |
|---|---|
| 6.1 | 6.43 |
| 7.7 | 5.40 |
| 11.4 | 53.62 |
| 11.9 | 33.37 |
| 14.2 | 8.03 |
| 16.5 | 6.70 |
| 18.3 | 13.86 |
| 19.0 | 6.68 |
| 20.7 | 10.02 |
| 21.3 | 7.02 |
| 22.8 | 24.68 |
| 24.0 | 100.00 |
| 28.3 | 33.26 |
| 32.7 | 6.35 |
| 36.0 | 3.28 |
| 38.5 | 6.02 |

In some embodiments, the crystalline glycolate salt of sepiapterin is characterized by peaks in the X-ray diffraction diagram observed at an angle of refraction 2θ at least at 7.6°±0.5, e.g., 7.6°±0.2, 10.7°±0.5, e.g., 10.7°±0.2, and 24.0°±0.5, e.g., 24.0°±0.2. In some embodiments, the most intense peak in the X-ray diffraction diagram is observed at an angle of refraction 2θ of 7.6°±0.5, e.g., 7.6°±0.2. In an essentially pure material of the crystalline glycolate salt of sepiapterin, peaks can be observed at angles of refraction 2θ as set forth in Table 20. Alternatively or in addition, the crystalline glycolate salt of sepiapterin is characterized by a DSC curve showing two endotherms at 133.9° C and 147.7° C.

**Table 20**

| **Position [2θ°] (±0.5, e.g., ±0.2)** | **Relative Intensity** |
|---|---|
| 4.8 | 6.23 |
| 7.6 | 100.00 |
| 10.3 | 68.06 |
| 10.7 | 70.69 |
| 15.3 | 36.51 |
| 18.2 | 24.25 |
| 18.7 | 27.26 |
| 19.9 | 2.66 |
| 21.2 | 17.11 |
| 24.0 | 96.62 |
| 24.4 | 18.44 |
| 28.8 | 47.57 |
| 30.3 | 7.43 |
| 32.5 | 4.42 |
| 33.3 | 7.49 |
| 34.3 | 5.21 |
| 36.3 | 7.37 |

In some embodiments, the crystalline acetate salt of sepiapterin is characterized by peaks in the X-ray diffraction diagram observed at an angle of refraction 2θ at least at 6.2°±0.5, e.g., 6.2°±0.2, 12.0°±0.5, e.g., 12.0°±0.2, and 18.1°±0.5, e.g., 18.1°±0.2. In some embodiments, the most intense peak in the X-ray diffraction diagram is observed at an angle of refraction 2θ of at least 6.2°±0.5, e.g., 6.2°±0.2. In an essentially pure material of the crystalline acetate salt of sepiapterin, peaks can be observed at angles of refraction 2θ as set forth in Table 21. Alternatively or in addition, the crystalline acetate salt of sepiapterin is characterized by a DSC curve showing two endotherms at 146.1° C and 175.4° C.

**Table 21**

| **Position [2θ°] (±0.5, e.g., ±0.2)** | **Relative Intensity** |
|---|---|
| 6.2 | 100.00 |
| 10.2 | 23.29 |
| 12.0 | 71.59 |
| 18.1 | 31.27 |
| 21.1 | 20.29 |
| 24.2 | 14.92 |
| 25.2 | 23.03 |
| 27.3 | 13.30 |
| 29.1 | 12.95 |

In some embodiments, the crystalline Form 1 sulfate salt of sepiapterin is characterized by peaks in the X-ray diffraction diagram observed at an angle of refraction 2θ at least at 5.1°±0.5, e.g., 5.1°±0.2, 7.8°±0.5, e.g., 7.8°±0.2, and 23.0°±0.5, e.g., 23.0°±0.2. In some embodiments, the most intense peak in the X-ray diffraction diagram is observed at an angle of refraction 2θ of 5.1°±0.5, e.g., 5.1°±0.2. In an essentially pure material of the crystalline Form 1 sulfate salt of sepiapterin, peaks can be observed at angles of refraction 2θ as set forth in Table 22. Alternatively or in addition, the crystalline form 1 sulfate salt of sepiapterin is characterized by a DSC curve showing three endotherms at 94.5° C, 158.3° C, and 209.9° C.

**Table 22**

| **Position [2θ°] (±0.5, e.g., ±0.2)** | **Relative Intensity** |
|---|---|
| 5.1 | 100.00 |
| 6.8 | 3.33 |
| 7.8 | 43.48 |
| 10.2 | 15.92 |
| 15.7 | 18.13 |
| 17.2 | 8.33 |
| 18.7 | 6.49 |
| 19.8 | 5.19 |
| 21.3 | 5.52 |
| 23.0 | 19.05 |
| 23.5 | 8.29 |
| 24.2 | 5.59 |
| 24.8 | 17.44 |
| 25.7 | 4.97 |
| 26.7 | 10.38 |
| 28.7 | 11.49 |
| 30.4 | 2.88 |
| 31.0 | 3.67 |

In some embodiments, the crystalline Form 2 sulfate salt of sepiapterin is characterized by peaks in the X-ray diffraction diagram observed at an angle of refraction 2θ at least at 7.8°±0.5, e.g., 7.8°±0.2, 8.8°±0.5, e.g., 8.8°±0.2, and 24.1°±0.5, e.g., 24.1°±0.2. In some embodiments, the most intense peak in the X-ray diffraction diagram is observed at an angle of refraction 2θ of 8.8°±0.5, e.g., 8.8°±0.2. In an essentially pure material of the crystalline Form 2 sulfate salt of sepiapterin, peaks can be observed at angles of refraction 2θ as set forth in Table 23.

**Table 23**

| **Position [2θ°] (±0.5, e.g., ±0.2)** | **Relative Intensity** |
|---|---|
| 5.0 | 4.71 |
| 7.9 | 72.24 |
| 8.8 | 100.00 |
| 14.5 | 19.26 |
| 15.7 | 59.40 |
| 16.1 | 8.69 |
| 17.2 | 14.82 |
| 17.7 | 10.89 |
| 19.3 | 9.92 |
| 20.2 | 9.60 |
| 23.7 | 15.38 |
| 24.2 | 43.88 |
| 25.0 | 11.44 |
| 26.8 | 16.81 |
| 28.7 | 16.07 |
| 29.4 | 13.84 |
| 31.3 | 17.14 |
| 31.7 | 7.26 |
| 35.7 | 5.75 |

The methods described herein may employ a pharmaceutical composition including a pharmaceutically acceptable excipient and an effective amount of sepiapterin, or a pharmaceutically acceptable salt thereof. Examples of pharmaceutical compositions of sepiapterin and salts thereof can be found in WO 2019/046849 and WO 2019/232120.

The pharmaceutically acceptable excipient can be any of those conventionally used and is limited only by chemico-physical considerations, such as solubility and by the route of administration. It will be appreciated by one of skill in the art that, in addition to the following described pharmaceutical compositions, sepiapterin can be formulated as inclusion complexes, such as cyclodextrin inclusion complexes, or liposomes.

The pharmaceutically acceptable excipients described herein, for example, vehicles, adjuvants, excipients, or diluents, are well known to those who are skilled in the art and are readily available to the public. It is preferred that the pharmaceutically acceptable excipient be one which is chemically inert to the sepiapterin and one which has no detrimental side effects or toxicity under the conditions of use.

### Formulations which increase gastric and/or anterior intestine residence time

Gastro-retentive drug delivery is an approach with the drug formulation is designed to remain in the stomach longer, e.g., until drug release is complete.

Bioadhesive dosage forms utilize polymers that are capable of adhering to surfaces and result in a controlled release of the drug. The bioadhesive polymers may be anionic (e.g., carboxymethylcellulose, alginic acid, polyacrylic acid, pectin, carrageenan, polycarbophil, or carbomer); cationic (e.g., chitosan, polylysine, or polybrene); or non-ionic (e.g., polyethylene glycol, polyvinylpyrrolidone, dextran, orhydroxypropylmethylcellulose).

High-density dosage forms are designed to sit in the stomach at a lower level than the pyloric sphincter, and thus avoid emptying. Excipients suitable for high-density dosage forms include iron powder, barium sulphate, zinc oxide, and titanium oxide.

Expandable dosage forms are designed to expand in the stomach to be larger than the pyloric sphincter, and thus avoid emptying. For example, dosage forms including a drug core, a swellable hydrocolloid, and an outer semi-permeable polymer are suitable for expandable dosage forms.

Super-porous hydrogel dosage forms are designed, similarly to expandable dosage forms, to expand in the stomach to be larger than the pyloric sphincter. Super-porous hydrogel dosage forms may include polymers such as cross-carmellose sodium.

Floating dosage forms are designed to have a lower density than gastric fluid. Floating dosage forms may include compositions including ion exchange resin, a raft system, an inflatable chamber, an effervescent mixture, a swellable hydrocolloid, or a multi-particulate system.

### Antioxidants

Sepiapterin is prone to rapid oxidation when exposed to air. Accordingly, pharmaceutical compositions described herein may include antioxidants. The antioxidant may minimize the oxidative degradation of sepiapterin. Examples of antioxidants include, but are not limited to, ascorbic acid, tocopherol, retinol, ascorbyl palmitate, N-acetyl cysteine, glutathione, ethylenediaminetetraacetic acid, sodium bisulfite, sodium metabisulfite, thiourea, butylatedhydroxytoluene, butylatedhydroxyanisole, and vitamin E. In some embodiments, the pharmaceutical compositions described herein include ascorbic acid, tocopherol, retinol, ascorbyl palmitate, N-acetyl cysteine, glutathione, butylatedhydroxytoluene, and/or butylatedhydroxyanisole as antioxidant.

In some embodiments, the pharmaceutical composition includes less than 10% antioxidant by weight, e.g., less than 9%, less than 8%, less than 7%, less than 6%, less than 5%, less than 4%, less than 3%, less than 2%, less than 1%, or substantially free of antioxidant. In some embodiments, the pharmaceutical composition includes 2-9% antioxidant by total weight, e.g., 2-4%, 3-5%, 4-6%, 5-7%, 6-8%, or 7-9%. In some embodiments, the pharmaceutical composition comprises 5-100% of the USP maximum daily dose of the antioxidant, e.g., in some embodiments, the pharmaceutical composition comprises about 5%, 10%, 15%, 20%, 30%, 40%, 50%, 60%, 70%, 80%, 90%, or 100% of the USP maximum daily dose of the antioxidant. In some embodiments, the ratio of sepiapterin to antioxidant is at least 1:1, e.g., 2:1, 3:1, 4:1, 5:1, 6:1, 7:1, 8:1, 9:1, or 10:1 by weight.

### Dispersants

In some embodiments, the pharmaceutical compositions described herein include at least one dispersant. The dispersant may cause particles in the formulation to separate, e.g., release their medicinal substances on contact with moisture. Examples of dispersants include, but are not limited to, crosslinked polyvinylpyrrolidone, carboxymethylcellulose (e.g., croscarmellose salt, e.g., croscarmellose sodium), starch (e.g., sodium starch glycolate), or alginic acid. In some embodiments, the dispersant in the pharmaceutical composition is a carboxymethylcellulose such as a pharmaceutically acceptable salt of croscarmellose. In some embodiments, the pharmaceutical composition may include 0.1-1.5% dispersant by total weight, e.g., about 0.1%, 0.5%, 1%, or 1.5%. In some embodiments, the pharmaceutical composition includes less than 1.5% dispersant, e.g., less than 1%, less than 0.5%, or less than 0.1% by total weight.

### Anti-caking agents

In some embodiments, the pharmaceutical compositions described herein include at least one anti-caking agent. In some embodiments, the pharmaceutical compositions include at least two anti-caking agents. Exemplary anti-caking agents include colloidal silicon dioxide, microcrystalline cellulose, tricalcium phosphate, microcrystalline cellulose, magnesium stearate, sodium bicarbonate, sodium ferrocyanide, potassium ferrocyanide, calcium ferrocyanide, calcium phosphate, sodium silicate, colloidal silicon dioxide, calcium silicate, magnesium trisilicate, talcum powder, sodium aluminosilicate, potassium aluminum silicate, calcium aluminosilicate, bentonite, aluminum silicate, stearic acid, and polydimethylsiloxane. In some embodiments, the at least one anti-caking agent is colloidal silicon dioxide or microcrystalline cellulose. In some embodiments, the pharmaceutical composition may include 65-75% anti-caking agent by total weight, e.g., about 65%, 67%, 70%, 73%, or 75%. In some embodiments, the pharmaceutical composition includes both colloidal silicon dioxide and microcrystalline cellulose. In some embodiments, the pharmaceutical composition includes 60-65% microcrystalline cellulose by total weight and 5-7% colloidal silicon dioxide by total weight.

### Dosing vehicle

In some embodiments, the pharmaceutical compositions described herein are combined with a dosing vehicle prior to administration, e.g., a dosing vehicle with a viscosity of approximately 50-1750 centipoise (cP). One type of suspending agent that can be used is a combination of glycerin and sucrose in water (e.g., MEDISCA^{®} oral mix with 2.5% glycerin and 27% sucrose in water). An appropriate quantity of composition can be added to the dosing vehicle mixture and agitated to suspend the composition just prior to administration.

Other suspending agents may also be used as a dosing vehicle. Exemplary suspending agents include agar, alginic acid, sodium carboxymethyl cellulose, carrageenan, dextrin, gelatin, guar gum, hydroxyethyl cellulose, hydroxypropyl cellulose, hypromellose, methyl cellulose, polyethylene glycol, povidone, tragacanth, xanthan gum, or other suspending agents known in the art.

### Dosage

Sepiapterin, or pharmaceutically acceptable salt thereof, can be used in any suitable dose. Suitable doses and dosage regimens can be determined by conventional range finding techniques. Generally treatment is initiated with smaller dosages, which are less than the optimum dose. Thereafter, the dosage is increased by small increments until optimum effect under the circumstances is reached. For convenience, the total daily dosage may be divided and administered in portions during the day if desired. In proper doses and with suitable administration, a wide range of responses are provided. Typically, the dosages range from about 2.5 to about 150 mg/kg body weight of the subject being treated/day, e.g., 60 mg/kg/day. For example, in embodiments, sepiapterin, or pharmaceutically acceptable salt thereof, may be administered from about 20 mg/kg to about 150 mg/kg, from about 20 mg/kg to about 60 mg/kg, from about 40 mg/kg to about 100 mg/kg, from about 100 mg/kg to about 150 mg/kg, from about 60 mg/kg to about 120 mg/kg, from about 80 mg/kg to about 100 mg/kg, from about 40 mg/kg to about 60 mg/kg, from about 2.5 mg/kg to about 20 mg/kg, from about 2.5 mg/kg to about 10 mg/kg, or from about 2.5 mg/kg to about 5 mg/kg, of subject body weight per day, one or more times a day, to obtain the desired therapeutic effect.

In some embodiments, the sepiapterin, or pharmaceutically acceptable salt thereof, can be formulated into unit solid oral dosage forms such as particles. In these embodiments, each unit solid oral dosage form, e.g., sachet, can comprise any suitable amount of the sepiapterin, or pharmaceutically acceptable salt thereof. For example, each solid oral dosage form can comprise about 2.5 mg, about 5 mg, about 10 mg, about 20 mg, about 30 mg, about 40 mg, about 50 mg, about 60 mg, about 70 mg, about 80 mg, about 90 mg, about 100 mg, about 125 mg, about 150 mg, about 175 mg, about 200 mg, about 225 mg, about 250 mg, about 275 mg, about 300 mg, about 325 mg, about 350 mg, about 375 mg, about 400 mg, about 425 mg, about 450 mg, about 475 mg, about 500 mg, about 750 mg, about 1 g, about 1.25 g, or about 1.5 g.

Sepiapterin, or a pharmaceutically acceptable salt thereof, can be used in the preparation of liquid formulations, such as in the form of a solution, suspension, or emulsion. Formulations suitable for oral administration include, but are not limited to, (a) capsules, sachets, tablets, lozenges, and troches, each containing a predetermined amount of the active ingredient, as solids or granules; (b) powders; (c) liquid solutions, such as an effective amount of the compound dissolved in diluents, such as water, saline, or orange juice; (d) suspensions in an appropriate liquid; and (e) suitable emulsions. Preferred are solid oral dosage forms such as capsule forms, tablet forms, and powder forms. Capsule forms can be of the ordinary hard- or soft-shelled gelatin type containing, for example, surfactants, lubricants, and inert fillers, such as lactose, sucrose, calcium phosphate, and cornstarch. Tablet forms can include one or more of lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible excipients. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, gels, and the like containing, in addition to the active ingredient, such excipients as are known in the art.

Formulations suitable for oral and/or parenteral administration include aqueous and non-aqueous, isotonic sterile injection solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in a physiologically acceptable diluent in a pharmaceutical excipient, such as a sterile liquid or mixture of liquids, including water, saline, aqueous dextrose and related sugar solutions, an alcohol, such as ethanol, benzyl alcohol, or hexadecyl alcohol, glycols, such as propylene glycol or polyethylene glycol and other polyethylene alcohols, glycerol ketals, such as 2,2-dimethyl-1,3-dioxolane-4-methanol, ethers, such as poly(ethylene glycol) 400, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropylmethylcellulose, or carboxymethylcellulose, or emulsifying agents and other pharmaceutical adjuvants.

Also described herein are pharmaceutical compositions in an orally tolerable formula that contains a therapeutically effective amount of sepiapterin and less than 10% antioxidant. In some embodiments, the pharmaceutical composition is a granular formulation that is dispersed in a pharmaceutically acceptable excipient, for example the composition can be mixed into water and ingested by a subject (e.g., over the course of 5 to 10 minutes). Suitable formulations are found in Remington's Pharmaceutical Sciences, Mack Publishing Company, Philadelphia, PA 22nd ed., 2010. Except insofar as any conventional excipient is incompatible with the active ingredient, its use in the pharmaceutical compositions is contemplated. Moreover, for animal (e.g., human) administration, it will be understood that preparations should meet sterility, pyrogenicity, general safety and purity standards as required by FDA Office of Biological Standards.

### Solid dosage form for oral administration

Formulations for oral use include particles containing the active ingredient(s) in a mixture with non-toxic pharmaceutically acceptable excipients, and such formulations are known to the skilled artisan (e.g., U.S. Patent Nos.: 5,817,307, 5,824,300, 5,830,456, 5,846,526, 5,882,640, 5,910,304, 6,036,949, 6,036,949, 6,372,218). Excipients may be, for example, inert diluents or fillers (e.g., sucrose, sorbitol, sugar, mannitol, microcrystalline cellulose, starches including potato starch, calcium carbonate, sodium chloride, lactose, calcium phosphate, calcium sulfate, or sodium phosphate); granulating and disintegrating agents (e.g., cellulose derivatives including microcrystalline cellulose, starches including potato starch, croscarmellose sodium, alginates, or alginic acid); binding agents (e.g., sucrose, glucose, sorbitol, acacia, alginic acid, sodium alginate, gelatin, starch, pregelatinized starch, microcrystalline cellulose, magnesium aluminum silicate, carboxymethylcellulose sodium, methylcellulose, hydroxypropyl methylcellulose, ethylcellulose, polyvinylpyrrolidone, or polyethylene glycol); and lubricating agents, glidants, anti-adhesives (e.g., magnesium stearate, zinc stearate, stearic acid, silicas, hydrogenated vegetable oils, or talc), and anti-caking agents (e.g., colloidal silicon dioxide, microcrystalline cellulose, tricalcium phosphate, microcrystalline cellulose, magnesium stearate, sodium bicarbonate, sodium ferrocyanide, potassium ferrocyanide, calcium ferrocyanide, calcium phosphate, sodium silicate, colloidal silicon dioxide, calcium silicate, magnesium trisilicate, talcum powder, sodium aluminosilicate, potassium aluminum silicate, calcium aluminosilicate, bentonite, aluminum silicate, stearic acid, polydimethylsiloxane). Other pharmaceutically acceptable excipients can be colorants, flavoring agents, plasticizers, humectants, and buffering agents. In some embodiments, excipients (e.g., flavoring agents) are packaged with the composition. In some embodiments, excipients (e.g., flavorings) are packaged separately from the composition (e.g., are combined with the composition prior to administration).

The solid compositions described herein may include a coating adapted to protect the composition from unwanted chemical changes, (e.g., chemical degradation prior to the release of the active substances). The coating may be applied on the solid dosage form in a similar manner as that described in *Encyclopedia of Pharmaceutical Technology,* supra.

Powders and granulates may be prepared using the ingredients mentioned above in a conventional manner using, e.g., a mixer, a fluid bed apparatus, melt congeal apparatus, rotor granulator, extrusion/spheronizer, or spray drying equipment.

### Methods of Treatment

Sepiapterin may serve as a useful therapeutic for diseases associated with increased blood phenylalanine concentrations, such as hyperphenylalaninemia or phenylketonuria. As described herein, various forms of these diseases may be treated, e.g., hyperphenylalaninemia caused by phenylketonuria, tetrahydrobiopterin-responsive phenylketonuria, sepiapterin-responsive phenylketonuria, classical phenylketonuria, or non-classical phenylketonuria. Thus, the various forms of sepiapterin, or a salt thereof, as described herein, can be administered to a subject in an effective amount to obtain a treatment or amelioration of the disease, disorder or condition.

Subjects treated by the methods herein have a blood phenylalanine concentration (e.g., an uncontrolled phenylalanine blood concentration) of greater than 120 micromole per liter, (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter). Subjects may also be stratified by having a blood phenylalanine concentration (e.g., an uncontrolled phenylalanine blood concentration) of between 120 and 360 micromole per liter, between 360 and 600 micromole per liter, between 600 and 1200 micromole per liter, or greater than 1200 micromole per liter.

Subjects having insufficient reduction in blood phenylalanine under existing therapy (as defined in the appended claims) may also be treated by the methods described herein. For example, a subject treated by therapy other than sepiapterin may have a blood phenylalanine concentration of greater than 120 micromole per liter (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) on existing management.

Administering sepiapterin, or a pharmaceutically acceptable salt thereof, may reduce the blood phenylalanine concentration of a subject to less than 600 micromole per liter, e.g., less than 360 micromole per liter or less than 120 micromole per liter, such as between 120 and 360 micromole per liter or between 360 and 600 micromole per liter. Alternatively or in addition, administering sepiapterin may reduce the blood phenylalanine concentration, e.g., prior to administration of sepiapterin, of the subject by at least 10% (e.g., at least 20%, at least 30%, at least 40%, at least 50%, at least 60%, at least 70%, at least 80%, at least 90% or from about 10% to about 30%, from about 20% to about 40%, from about 30% to about 50%, from about 40% to about 60%, from about 50% to about 70%, from about 60% to about 80%, or about 70% to about 90%). The reduction may be determined after administration over at least 1 week (e.g., at 2 weeks, 3 weeks, 4 weeks, 5 weeks, 6 weeks, at least about two weeks, at least about three weeks, at least about four weeks, at least about 5 weeks, at least about 6 weeks). Administering sepiapterin may also produce a BH4 concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration.

In the methods described herein, the subject has failed to respond to treatment with sapropterin, or a pharmaceutically acceptable salt thereof (e.g., sapropterin dihydrochloride). The subject may have additionally failed to respond to treatment with pegvaliase-pqpz. For example, the blood plasma concentration of the subject may have decreased less than 20% upon administration of pegvaliase-pqpz. For example, the blood plasma concentration of the subject decreased less than 20% after administration of at least about 20 mg once daily of pegvaliase-pqpz for at least eight days (e.g., at least fourteen days, at least 21 days, at least 28 days, at least 30 days). In the methods described herein, the blood plasma concentration of the subject decreased less than 20% upon administration, e.g., at least about 10 mg/kg (e.g., at least about 15 mg/kg, at least about 20 mg/kg), of sapropterin, or a pharmaceutically acceptable salt thereof (e.g., sapropterin dihydrochloride). In some embodiments, the blood plasma concentration of the subject decreased less than 15% upon administration, e.g., at least about 10 mg/kg (e.g., at least about 15 mg/kg, at least about 20 mg/kg), of sapropterin, or a pharmaceutically acceptable salt thereof (e.g., sapropterin dihydrochloride). In some embodiments, the blood plasma concentration of the subject was greater than 120 micromole per liter, (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) after administration of sapropterin, or a pharmaceutically acceptable salt thereof, (e.g., sapropterin dihydrochloride) for at least eight days (e.g., at least fourteen days, at least 21 days, at least 28 days, at least 30 days) or at least about 20 mg once daily of pegvaliase-pqpz for at least sixteen weeks (e.g., at least eighteen weeks, at least 20 weeks, at least 22 weeks, at least 24 weeks). For example, the blood plasma concentration of the subject was greater than 120 micromole per liter, (e.g., greater than 200 micromole per liter, greater than 300 micromole per liter, greater than 360 micromole per liter, greater than 400 micromole per liter, greater than 450 micromole per liter, greater than 500 micromole per liter, greater than 550 micromole per liter, greater than 600 micromole per liter, greater than 650 micromole per liter, greater than 700 micromole per liter, greater than 800 micromole per liter, greater than 900 micromole per liter, greater than 1000 micromole per liter, greater than 1100 micromole per liter, or greater than 1200 micromole per liter) after administration of at least about 10 mg/kg (e.g., at least about 15 mg/kg, at least about 20 mg/kg) of sapropterin, or a pharmaceutically acceptable salt thereof, (e.g., sapropterin dihydrochloride) for at least eight days (e.g., at least fourteen days, at least 21 days, at least 28 days, at least 30 days) or at least about 20 mg once daily of pegvaliase-pqpz for at least sixteen weeks (e.g., at least eighteen weeks, at least 20 weeks, at least 22 weeks, at least 24 weeks).

The subject may also be administered sepiapterin after discontinuing treatment with pegvaliase-pqpz due to an adverse reaction (e.g., anaphylaxis, an injection site reaction, arthralgia, a hypersensitivity reaction, headache, a generalized skin reaction lasting at least 14 days, pruritus, nausea, abdominal pain, oropharyngeal pain, vomiting, cough, diarrhea, and/or fatigue). The risk of adverse events (e.g., headache, rhinorrhea, pharyngolaryngeal pain, diarrhea, vomiting, cough, and/or nasal congestion) may be reduced compared to a subject administered at least 10 mg/kg (e.g., at least 15 mg/kg, at least 20 mg/kg) sapropterin, or a pharmaceutically acceptable salt thereof (e.g., sapropterin dihydrochloride) and/or tolerability.

In some embodiments, the subject is on a phenylalanine-restricted diet (e.g., the subject is on a diet including a milk substitute or formula such as Phenyl-Free 2 and measured amounts of fruits, vegetables, bread, pasta, and cereals). In some embodiments, the subject is not on a phenylalanine-restricted diet (e.g., the subject is not on a diet including a milk substitute or formula such as Phenyl-Free 2 and measured amounts of fruits, vegetables, bread, pasta, and cereals). In some embodiments, the subject has a median phenylalanine intake of less than 3000 mg/day (e.g., less than 2500 mg/day, less than 2000 mg/day, less than 1500 mg/day, less than 1000 mg/day, less than 500 mg/day). Treatment with sepiapterin may allow a reduction in blood phenylalanine concentration (e.g., below 600, 360, or 120 micromole/liter) in conjunction with phenylalanine consumption or consumption of natural protein. For example, a subject may consume phenylalanine at at least about 1000 mg/day (e.g., at least about 1100 mg/day, at least about 1200 mg/day, at least about 1300 mg/day, at least about 1400 mg/day, at least about 1500 mg/day, at least about 1600 mg/day, at least about 1700 mg/day, at least about 1800 mg/day, at least about 1900 mg/day, or at least about 2000 mg/day or from 1000 mg/day to 1400 mg/day, from 1200 mg/day to 1600 mg/day, from 1300 mg/day to 1700 mg/day, from 1600 mg/day to 2000 mg/day, from 1800 mg/day to 2400 mg/day, from 2000 mg/day to 3000 mg/day, from 3000 mg/day to 4000 mg/day, from 4000 to 5000 mg/day). In some embodiments, less than 25% (e.g., less than 20%, less than 15%, less than 10%, or less than 5%) of the protein in the subject's diet is natural protein or more than 25% (e.g., more than 30%, more than 40%, more than 50%, more than 60%, more than 70%, more than 80%, more than 90%, more than 95%, more than 99%) of the protein in the subject's diet is natural protein. In some embodiments, the subject has a natural protein intake of greater than 10 g/day (e.g., greater than 20 g/day, greater than 30 g/day, greater than 40 g/day, greater than 50 g/day, greater than 60 g/day, greater than 70 g/day, greater than 80 g/day or from about 10 g/day to about 30 g/day, about 20 g/day to about 40 g/day, about 30 g/day to about 50 g/day, about 40 g/day to about 60 g/day, about 50 g/day to about 70 g/day, about 60 g/day to about 80 g/day). In some embodiments of any of the methods described herein, the subject has a natural protein intake of less than 10 g/day (e.g., less than 5 g/day or from about 5 g/day to about 10 g/day).

In some embodiments, the subject has PKU defined as any prior blood Phe measurement >360 µmol/L plus at least one measurement >450 µmol/L, or an average of the 3 most recent blood Phe measurements >450 µmol/L. In some embodiments, the subject does not have PKU defined as any prior blood Phe measurement >360 µmol/L plus at least one measurement >450 µmol/L, or an average of the 3 most recent blood Phe measurements >450 µmol/L. In some embodiments, the subject has classical PKU defined as any documented historical measurement of blood Phe of at least 1,200 µmol/L. In some embodiments, the subject does not have classic PKU classical PKU defined as any documented historical measurement of blood Phe of at least 1,200 µmol/L.

In some embodiments, the subject is a child (e.g., the subject is less than 18 years old, less than 17 years old, less than 16 years old, less than 15 years old, less than 14 years old, less than 13 years old, less than 12 years old, less than 11 years old, less than 10 years old, less than 9 years old, less than 8 years old, less than 7 years old, less than 6 years old, less than 5 years old, less than 4 years old, less than 3 years old, less than 2 years old, less than 1 year old). In some embodiments, the subject is an adult (e.g., the subject is greater than 18 years old).

In some embodiments the treatment may increase neurocognitive function of the subject (e.g., an increase in executive function, a decrease in anxiety, a decrease in attentiondeficit/hyperactivity disorder symptoms, and/or a decrease in instances of brain fog). Changes in executive function may be determined by an assessment, such as Behavioural Assessment of Dysexecutive Syndrome (BADS), Behavior Rating Inventory of Executive Function (BRIEF or Mini-BRIEF), Barkley Deficits in Executive Functioning Scales (BDEFS), Behavioral Dyscontrol Scale (BDS), the ASEBA Child Behavior Checklist (CBLC), Comprehensive Executive Function Inventory (CEFI), CogScreen, Continuous Performance Task (CPT), Controlled Oral Word Association Test (COWAT), d2 Test of Attention, Delis-Kaplan Executive Function System (D-KEFS), Digit Vigilance Test, Figural Fluency Test, Halstead Category Test, Hayling and Brixton tests, lowa gambling task, Kaplan Baycrest Neurocognitive Assessment (KBNA), Kaufman Short Neuropsychological Assessment, Mental Clutter Scale, Paced Auditory Serial Addition Test (PASAT), phenylketonuria - quality of life (PKU-QOL), Rey-Osterrieth Complex Figure, Ruff Figural Fluency Test, Stroop task, Tasks of Executive Control, Test of Variables of Attention (T.O.V.A.), Tower of London Test, Trail-Making Test (TMT) or Trails A & B, Wisconsin Card Sorting Test (WCST), Symbol Digit Modalities Test, or a Cambridge Neuropsychological Test Automated Battery (CANTAB) assessment, e.g., by measuring reaction time, spatial span, spatial working memory, rapid digital information processing, sustained attention, and/or stop signal task. The treatment may produce an improvement in attention and/or mood, e.g., as measured by the ADHD-RS 5 scale (or the Inattention assessment thereof) and/or the Profile Mood States (POMS) scale. The treatment may produce an increase in sleep quality and/or a decrease in symptoms associated with sleep deprivation.

Sepiapterin may or may not be administered with food. Without being bound by theory, administration of sepiapterin with food results in an increase in plasma exposure of BH4, e.g., by reducing the rate of absorption of sepiapterin. If the administered sepiapterin is absorbed quickly, e.g., by being administered on an empty stomach, sepiapterin reductase and/or dihydrofolate reductase in the cells may become saturated above Vₘₐₓ resulting in at least a portion of the administered sepiapterin leaving the cell without being reduced to 7,8-dihydrobiopterin and subsequently to BH4. This excess sepiapterin may then be excreted without ever being converted to BH4, resulting in lower levels of BH4 in the plasma compared to administration of sepiapterin with food which reduces the rate of or prolongs the absorption of sepiapterin and results in reaction rates below, at or slightly above the Vₘₐₓ for substrate saturation of sepiapterin reductase enzyme and/or dihydrofolate reductase. Administration of sepiapterin with food unexpectedly results in an increase in the maximum BH4 plasma concentration (Cmax) and the extent of exposure as measured by the area under the concentration time curve of time zero to last concentration (AUC₀₋ₗₐₛₜ) of BH4 compared to administration without food. For example, the effective amount of sepiapterin is an amount (e.g., 2.5 mg/kg to 100 mg/kg per dose) sufficient to produce a BH4 concentration of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL, from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration with food. The effective amount may include a dose that is at least 5% (at least 10%, at least 20%, at least 50%, at least 70%, at least 90%, at least 100%, at least 110%, at least 120%, at least 130%, at least 140%, or at least 150%) lower than the dose sufficient to produce a maximum BH4 plasma concentration (Cmax) of at least 50 ng/mL (e.g., at least 60 ng/mL, at least 100 ng/mL, at least 200 ng/mL, at least 400 ng/mL, at least 600 ng/mL, at least 1000 ng/mL, or at least 2000 ng/mL, or from 50 ng/mL to 100 ng/mL from 60 ng/mL to 400 ng/mL, from 200 ng/mL to 600 ng/mL, from 400 ng/mL to 1000 ng/mL, or from 600 ng/mL to 1500 ng/mL) in the plasma of the subject within 10 hours of administration of sepiapterin without food.

In some embodiments of any of the methods described herein, the food is a high protein food. In some embodiments of any of the methods described herein, the food is a high fat food (e.g., at least 25, 30, 40, or 50% of the calories are from fat). In some embodiments of any of the methods described herein, the food is a high protein and high fat food. In some embodiments, the food is high calorie food (e.g., the food includes at least 100 calories, e.g., at least 200 calories, at least 300 calories, at least 400 calories, at least 500 calories, e.g., 500-1500 or 800-1000 calories). In some embodiments of any of the methods described herein, the food is a meal, e.g., breakfast, lunch, or dinner. The sepiapterin may be provided in a separate composition from the consumed food (e.g., the sepiapterin is not incorporated into a food product).

Administration to the subject may occur less than 30 minutes prior to consuming food or after consuming food, e.g., immediately prior to the consumption of food up to 1 hour after consumption, such as substantially at the same time as food. The administration with food (e.g., occurring less than 30 minutes prior to consuming food or after consuming food, e.g., immediately prior to the consumption of food up to 1 hour after consumption) may result in an increase (e.g., at least 5%, at least 10%, at least 20%, at least 30%, at least 40%, at least 50%, at least 60%, at least 70%, at least 80%, at least 90%, at least 100%, at least 110%, at least 120%, at least 130%, at least 140%, or at least 150%) in the Cmax of BH4 or in the extent of production and resulting plasma exposure (AUC₀₋ₗₐₛₜ) of BH4 compared to administration without food (e.g., occurring more than 2 hours after consuming food until 30 minutes prior to consuming further food).

The actual dosage amount of a composition described herein administered to a subject can be determined by physical and physiological factors such as body weight, severity of condition, the type of disease being treated, previous or concurrent therapeutic interventions, idiopathy of the subject and on the route of administration. Depending upon the dosage and the route of administration, the number of administrations of a preferred dosage and/or an effective amount may vary according to the response of the subject. The practitioner responsible for administration will, in any event, determine the concentration of active ingredient(s) in a composition and appropriate dose(s) for the individual subject.

In some embodiments, subjects receive about 2.5 mg/kg to 120 mg/kg per dose (e.g., about 20 mg/kg to about 60 mg/kg, or about 20 mg/kg, about 30 mg/kg, about 40 mg/kg, about 50 mg/kg, about 60 mg/kg). Subjects may receive the pharmaceutical composition including sepiapterin once daily, twice daily or three times daily during treatment. In some embodiments, subjects continue their other current treatments for high phenylalanine blood concentrations (e.g., phenylalanine-restricted diet) except for BH4 supplementation (if they are taking BH4). In some embodiments, subjects may not be permitted to take any drugs known to inhibit folate synthesis (e.g., methotrexate, pemetrexed, or trimetrexate). Sepiapterin, or a pharmaceutically acceptable salt thereof, may be administered in two equal doses (e.g., two doses at different times of day), e.g., two 60 mg/kg doses (e.g., one 60 mg/kg dose in the morning and one 60 mg/kg dose in the evening), two 40 mg/kg doses (e.g., one 40 mg/kg dose in the morning and one 40 mg/kg dose in the evening), two 30 mg/kg doses (e.g., one 30 mg/kg dose in the morning and one 30 mg/kg dose in the evening), two 20 mg/kg doses (e.g., one 20 mg/kg dose in the morning and one 20 mg/kg dose in the evening), or two 10 mg/kg doses (e.g., one 10 mg/kg dose in the morning and one 10 mg/kg dose in the evening).

In some embodiments, subjects who are taking BH4 discontinue administration of BH4 (i.e., BH4 washout, e.g., prior to or concomitant with initiation of sepiapterin treatment). Blood samples for Phe concentrations may be obtained during the BH4 washout period at 7, 5, 3, and 1 day before the treatment with the pharmaceutical composition described herein or until blood Phe levels are >360 µmol/L at any time point during BH4 washout. In some embodiments, pre-dose blood samples are tested for sepiapterin, Phe, BH4, and tyrosine (Tyr).

### EXAMPLES

### Example 1. Evaluation of the food effect on the administration of sepiapterin

Method: Subjects received 2 oral doses of sepiapterin (10 mg /kg) separated by 1 week in a fasted and fed state. Subjects were fed a standard high-fat (approximately 50 percent of total caloric content of the meal) and high-calorie (approximately 800 to 1000 calories) meal starting 30 minutes prior to receiving their second oral dose of sepiapterin on Day 8.

Sampling for PK analysis occurred predose on Days 1 and 8 (within 30 minutes before dosing) and 0.5 hr, 1 hr, 2 hr, 4 hr, 8 hr, 12 hr, and 24 hr after the doses on Days 1 and 8. The blood concentrations of sepiapterin and BH4 were analyzed by MNG Labs.

The cerebrospinal fluid (CSF) of selected subjects is collected via lumbar puncture on Day1 (before dose) and on Day 7 (i.e., following daily dosing for 7 days) approximately 30 minutes from time of the maximum observed plasma BH4 concentration (Tmax) as determined from the blood analysis.

The cerebral spinal fluid (CSF) was analyzed by MNG Labs. Descriptive statistics are provided to characterize any changes in neurotransmitter metabolism between the Day 1, and Day 7 sample results.

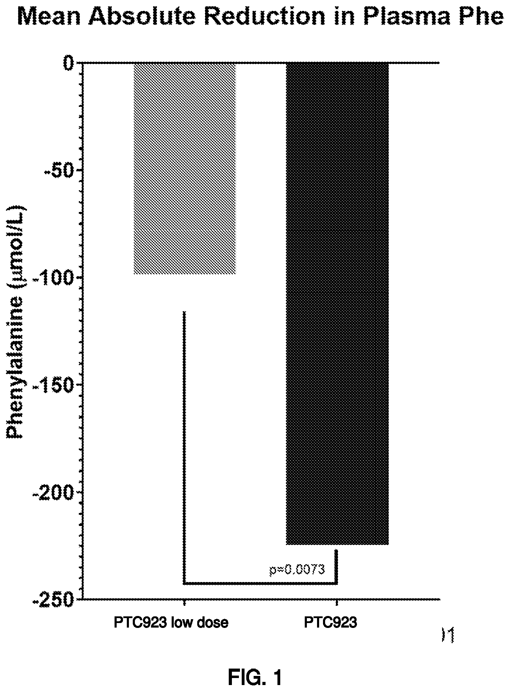

Results: As shown in Tables 24 and 25 below and FIG. 1, surprisingly, the Cmax of BH4 in the plasma was much higher in subjects when fed prior to administration compared to subjects who had fasted prior to administration. Further, there was a decrease in plasma sepiapterin concentration but an increase in BH4 concentration, when sepiapterin was administered in the fed vs. fasting state (FIG. 1). Geometric mean ratios (fasted/fed, 90%CI) for plasma sepiapterin were 1.29 (0.84 to 2.00) for AUCₗₐₛₜ and 1.57 (1.21 to 2.0) for Cₘₐₓ. Corresponding ratios (90%CI) for plasma BH4 were 0.58 (0.47 to 0.71) for AUC_{0-inf}, and 0.55 (0.45 to 0.68) for Cₘₐₓ. Overall exposure to BH4 as measured by AUC_{0-inf} and AUCₗₐₛₜ increased by 1.7-fold when sepiapterin was administered in the fed state compared with the fasted state.

**Table 24. Summary of BH4 Concentration in Plasma of Fasted Subjects**

| | Time (hours) | | | | | | | |
|---|---|---|---|---|---|---|---|---|
| | 0 | 0.5 | 1 | 2 | 4 | 8 | 12 | 24 |
| Mean (nM) | 7.62 | 25.65 | 154.63 | 413.48 | 624.34 | 248.09 | 85.87 | 18.33 |
| SD (nM) | 1.26 | 14.20 | 42.65 | 104.49 | 210.24 | 118.04 | 39.26 | 8.65 |
| Median (nM) | 7.75 | 22.85 | 147.30 | 370.40 | 635.05 | 232.55 | 75.30 | 15.35 |
| Minimum (nM) | 5.00 | 13.80 | 98.50 | 271.40 | 379.00 | 90.20 | 38.90 | 8.50 |
| Maximum (nM) | 9.00 | 66.50 | 223.10 | 617.90 | 1127.90 | 457.30 | 162.00 | 37.40 |

**Table 25. Summary of BH4 Concentration in Plasma of Fed Subjects**

| | Time (hours) | | | | | | | |
|---|---|---|---|---|---|---|---|---|
| | 0 | 0.5 | 1 | 2 | 4 | 8 | 12 | 24 |
| Mean (nM) | 8.43 | 33.32 | 165.79 | 586.90 | 1098.85 | 520.33 | 161.39 | 19.27 |
| SD (nM) | 1.45 | 21.70 | 82.02 | 249.26 | 289.74 | 192.50 | 58.43 | 8.20 |
| Median (nM) | 8.50 | 30.95 | 160.00 | 554.00 | 1010.60 | 485.00 | 142.30 | 16.15 |
| Minimum (nM) | 6.00 | 8.90 | 42.30 | 298.90 | 750.80 | 252.70 | 90.40 | 12.10 |
| Maximum (nM) | 11.00 | 76.40 | 369.00 | 1199.90 | 1566.70 | 926.30 | 261.10 | 40.10 |

Further, as shown in Tables 26 and 27 below, surprisingly, the Cmax of sepiapterin in the plasma was much lower in subjects when fed prior to administration compared to subjects who fasted prior to administration.

**Table 26. Summary of Sepiapterin Concentration in Plasma of Fasted Subjects**

| | Time (hours) | | | | | | | |
|---|---|---|---|---|---|---|---|---|
| | 0 | 0.5 | 1 | 2 | 4 | 8 | 12 | 24 |
| Mean (nM) | 0 | 2.85 | 5.43 | 4.63 | 1.88 | 0 | 0 | 0 |
| SD (nM) | 0 | 2.67 | 2.22 | 1.36 | 1.48 | 0 | 0 | 0 |
| Median (nM) | 0 | 2.32 | 4.82 | 4.71 | 2.37 | 0 | 0 | 0 |
| Minimum (nM) | 0 | 0 | 3.15 | 2.52 | 0 | 0 | 0 | 0 |
| Maximum (nM) | 0 | 7.23 | 10.19 | 6.65 | 3.93 | 0 | 0 | 0 |

**Table 27. Summary of Sepiapterin Concentration in Plasma of Fed Subjects**

| | Time (hours) | | | | | | | |
|---|---|---|---|---|---|---|---|---|
| | 0 | 0.5 | 1 | 2 | 4 | 8 | 12 | 24 |
| Mean (nM) | 0 | 0.63 | 2.38 | 2.96 | 2.96 | 0.18 | 0 | 0 |
| SD (nM) | 0 | 1.17 | 1.66 | 1.88 | 1.53 | 0.63 | 0 | 0 |
| Median (nM) | 0 | 0 | 2.42 | 2.91 | 2.50 | 0 | 0 | 0 |
| Minimum (nM) | 0 | 0 | 0 | 0 | 0 | 0 | 0 | 0 |
| Maximum (nM) | 0 | 3.22 | 4.57 | 5.92 | 5.56 | 2.19 | 0 | 0 |

### Example 2. Comparison of Adverse Events in Fed and Fasted Subjects

Method: Twelve subjects were given a single dose (10 mg/kg) of sepiapterin under fasting conditions and then 7 days later under fed conditions. Standard definitions of adverse events (AE) were used. All-cause AE were those that occurred at any time; treatment-emergent adverse events (TEAE) were those that occurred at or after the time of administration of study treatment. TEAEs related to study drug were based on the opinion of the investigator. Serious AE were defined as life threatening or caused death, hospitalization or prolongation of existing hospitalization, or were a persistent or significant disability/incapacity or a substantial disruption of the ability to conduct normal life functions, or a congenital anomaly/birth defect.

Results: As shown in Table 28, surprisingly, there was a decreased incidence of adverse events when sepiapterin was administered to fed subjects compared to fasted subjects.

**Table 28. Comparison of adverse events in fasted and fed conditions**

| | Sepiapterin 10 mg/kg | |
|---|---|---|
| | Fasted | Fed |
| ≥1 AE | 5 | 1 |
| TEAE | 4 | 1 |
| TEAE related to study drug | 1 | 0 |

### Example 3. Pharmacokinetic analysis of administration of multiple doses of sepiapterin

Methods: Three cohorts of eight fed subjects each were randomized to receive once-daily sepiapterin or placebo for 7 days in a 6:2 ratio. A sentinel dose strategy was also used for administration of the highest sepiapterin dose.

Results: Plasma-time concentrations of sepiapterin and BH4 were similar after 1 and 7 days of treatment with sepiapterin, with no drug accumulation. The pharmacokinetic data is shown in Table 29 below.

**Table 29. Summary of pharmacokinetic data for multiple administrations of sepiapterin**

| | | | **Dose of sepiapterin (mg/kg)** | | |
|---|---|---|---|---|---|
| **Parameter** | **Compound** | **Day** | **5** | **20** | **60** |
| Cₘₐₓ (ng/mL) | sepiapterin | 1 | 0.6 | 1.2 | 2.7 |
| | | 7 | 0.6 | 1.3 | 2.8 |
| | BH4 | 1 | 147 | 496 | 597 |
| | | 7 | 152 | 516 | 678 |
| Tₘₐₓ (h) | sepiapterin | 1 | 3.0 | 2.6 | 3.3 |
| | | 7 | 3.0 | 2.7 | 3.0 |
| | BH4 | 1 | 4.0 | 4.0 | 4.6 |
| | | 7 | 4.0 | 4.0 | 4.0 |
| AUC₀₋₂₄ (ng.h/mL) | sepiapterin | 1 | NC | NC | 23 |
| | | 7 | NC | NC | 23 |
| | BH4 | 1 | 994 | 3031 | 4560 |
| | | 7 | 1070 | 3718 | 4864 |
| AUC_{0-inf} (ng.h/mL) | sepiapterin | 1 | 1.0 | 3.9 | 21.2 |
| | | 7 | 0.7 | 4.3 | 16.0 |
| | BH4 | 1 | 1014 | 3085 | 4668 |
| | | 7 | NC | NC | NC |

| | | | | | |
|---|---|---|---|---|---|
| NC=Not calculated | | | | | |

### Example 4. Reduction of phenylalanine blood concentration in patients with PKU by administration of sepiapterin

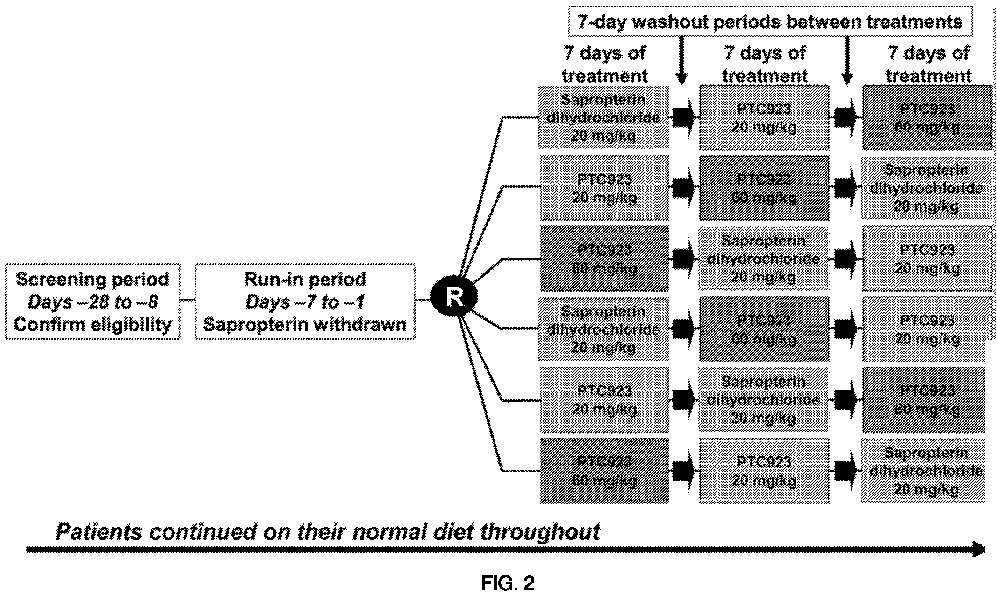

Following screening for eligibility, a 7-day run-in period commenced. Patients then received 7 days of once-daily oral treatment with either PTC923 20 mg/kg/day ("low dose"), or PTC923 60 mg/kg/day or sapropterin dihydrochloride, in a random order. PTC923 (formerly CNSA-001) is oral sepiapterin. Each patient was randomized to one of six treatment sequences (FIG. 2). The randomization in six different treatment sequences was intended to minimize carry over effect, treatment period effect, patient fatigue, and natural fluctuations in blood Phe levels. A 7-day washout period separated each active treatment period. Patients were asked to maintain their usual pre-study diet, including consumption of amino acid mixtures if prescribed, and were monitored throughout the study by a metabolic dietician.

Blood samples for measurement of Phe were taken on 7, 5, 3, and 1 days before randomization. During treatment periods, blood was taken pre-dose for day 1 and post-dose for days 3, 5 and 7. During the washout periods, blood was taken for measurement of Phe on days 1, 3, 5, and 7. All samples were taken under fasting conditions or no earlier than 3 hours following a meal and at the same time of day. Blood Phe was measured using a validated LC-MS-MS technique of dried blood sampling via Volumetric Absorptive Microsampling (VAMS^{®}) using Mitra^{®} 4-sampler clamshells, at a central bioanalytical laboratory (Agilex Biolabs Pty Ltd).

24 patients were randomized: all completed the study and were included in both the Efficacy and Safety populations. Patients' mean age was 26 y, and mean weight was 69 kg (Table 30). Twothirds of patients were women (67%). All patients were naïve to sapropterin treatment. Eleven out of twenty-four patients (46%) had classical PKU defined as any documented historical measurement of blood Phe of at least 1,200 µmol/L. Supplements including amino acids for the management of PKU were listed as received by 13 patients (54%) before the study and by 14 patients (58%) during the study

**Table 30. Patient demographics**

| | | |
|---|---|---|
| Mean age, years (SD) | | 26.0 (6.7) |
| Males/females, % | | 33/67 |
| Mean body weight, kg (SD) | | 69.2 (15.9) |

| Ethnicity, n (%) | | |
|---|---|---|
| | White | 24 (100) |
| | Non-Hispanic/Latino | 24 (100 |
| Naive to treatment with sapropterin, n (%) | | 24 (100) |

All patients entered the study on a low protein, Phe restricted diet and were instructed not to change their daily protein intake throughout the study. Mean [SD] daily dietary Phe intake was similar for the three groups at baseline (PTC923 60 mg/kg 2,550 [1,371] mg; PTC923 20 mg/kg 2,265 [946] mg; sapropterin dihydrochloride 20 mg/kg 2,510 [1,395] mg). Mean changes in daily dietary Phe intake to day 7 increased for all groups and were 19 [1,260] mg, 650 [1,262] mg, and 523 [1,132] mg, respectively.

PTC923 safety was monitored and adverse events (AEs) were coded using the Medical Dictionary for Regulatory Activities (MedDRA^{®}) version 21.0. Treatment-emergent adverse events (TEAEs) were defined as AE that began on or after first dose of study drug. A TEAE was associated with the study treatment currently being received, or the last study treatment before its onset. A treatment-related TEAE was, in the judgment of the investigator possibly, probably or definitely related to treatment. A serious AE (SAE) was defined as resulting in death, life-threatening AE, inpatient hospitalization or prolongation of existing hospitalization, a persistent or significant disability/incapacity or a substantial disruption of the ability to conduct normal life functions, or a congenital anomaly/birth defect.

Results: PTC923 at any dose reduced blood Phe significantly from baseline (Table 31). PTC923 60 mg/kg was significantly more effective than sapropterin dihydrochloride 20 mg/kg in reducing blood Phe (p=0.0098). The effect of PTC923 20 mg/kg on blood Phe was numerically larger than that of sapropterin dihydrochloride 20 mg/kg, As shown in FIG. 1, PTC923 low dose resulted in a mean absolute reduction in plasma phenylalanine of about 100 micromole per liter, and PTC923 resulted in a mean absolute reduction in plasma phenylalanine of over 200 micromole per liter.

**Table 31. Mean changes in blood Phe**

| | | | | | |
|---|---|---|---|---|---|
| | | | **PTC923** | | **Sapropterin dihydrochloride 20 mg/kg (n=24)** |
| | | | **60 mg/kg (n=24)** | **20 mg/kg (n=24)** | |

| **Mean (SD) blood Phe (µmol/L):** | | | | | |
|---|---|---|---|---|---|
| | **All subjects (N=24)** | | | | |
| | | At baseline | 727.8 (388.2) | 694.2 (396.0) | 710.4 (370.0) |
| | | On treatment^{a} | 517.7 (411.7) | 550.4 (433.7) | 619.5 (475.3) |
| | | Change | -210.1 (235.8) | -143.7 (224.8) | -90.8 (226.9) |
| Least squares mean (SE) change from baseline (µmol/L) | | | -206.4 (41.8) (p<0.0001) | -146.9 (41.8) (p=0.0010) | -91.5 (41.7) (p=0.0339) |

| | **Subjects with classical PKU (N=11)** 1) | | | | |
|---|---|---|---|---|---|
| | | At baseline | 947.2 (363.0) | 944.6 (354.6) | 903.5 (422.5) |
| | | On treatment^{a} | 801.2 (396.5) | 869.1 (424.1) | 914.2 (538.7) |
| | | Change | -146.0 (187.7) | -75.5 (201.8) | 10.7 (221.6) |
| Least squares mean (SE) change from baseline (µmol/L) | | | -150.8 (63.1) (p=0.0287) | -71.5 (61.8) (p=0.2629) | -2.8 (62.0) (p=0.9640) |

| | | | | | |
|---|---|---|---|---|---|
| ^{a}Mean of values measured on days 3, 5 and 7 of each treatment period (see text). | | | | | |

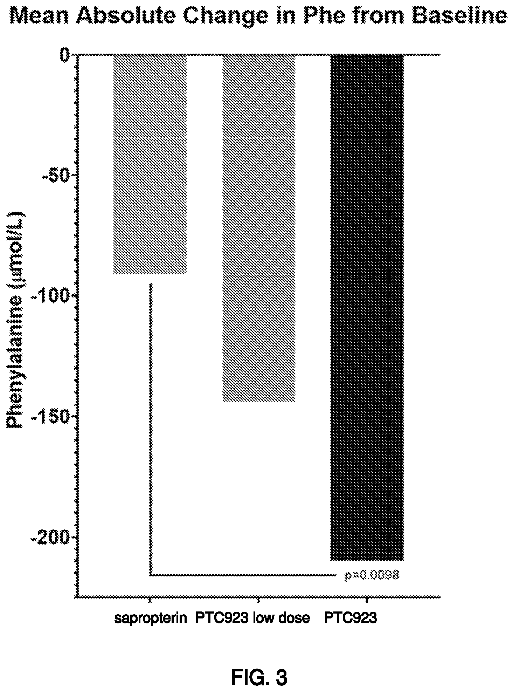

Least squares mean (LSM) changes (SE) from baseline blood Phe levels were: -206.4 (41.8) µmol/L for PTC923 60 mg/kg (p<0.0001), -146.9 (41.8) µmol/L for PTC923 20 mg/kg (p=0.0010) and -91.5 (41.7) µmol/L for sapropterin dihydrochloride (p=0.0339) (FIG. 3). The effect of PTC923 appeared to be dose-related.

The proportions of patients achieving blood Phe <360 µmol/L were 12/24 (50%) for patients randomized to PTC923 60 mg/kg, 11/24 (46%) for patients randomized to PTC923 20 mg/kg, and 10/24 (42%) for patients randomized to sapropterin dihydrochloride. The proportion of patients who achieved blood Phe <360 µmol/L on either dose of PTC923 was 13/24 (54%).

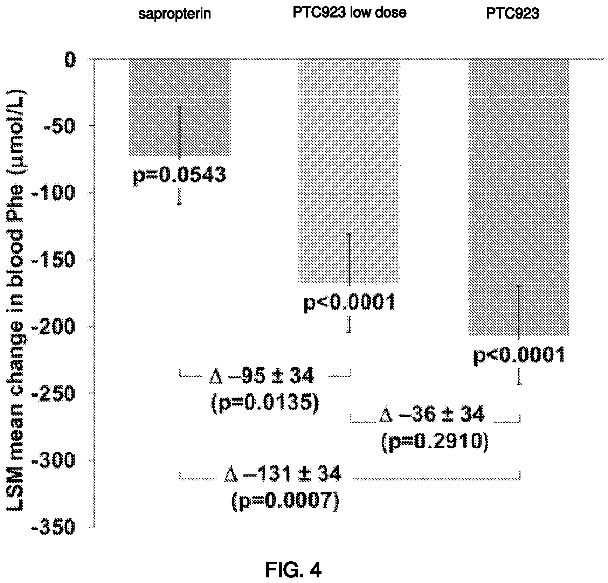

Blood Phe declined rapidly on study treatments. Least squares mean changes (SE; p value) from baseline in blood Phe to first Phe measurement after treatment initiation (Day 3) were: -206.6 (36.6; p<0.0001) µmol/L for PTC923 60 mg/kg; -167.5 (36.6; p<0.0001) µmol/L for PTC923 20 mg/kg; and -72.3 (36.6; p=0.0543) µmol/L for sapropterin dihydrochloride. The mean change in blood Phe reduction was significantly larger for both PTC923 doses vs. sapropterin dihydrochloride at Day 3 (LSM difference-131.3 [SE 33.8; p=0.0007] and -95.2 [SE 33.7; p=0.0135] for PTC923 60 mg/kg and PTC923 20 mg/kg, respectively) (FIG. 4).

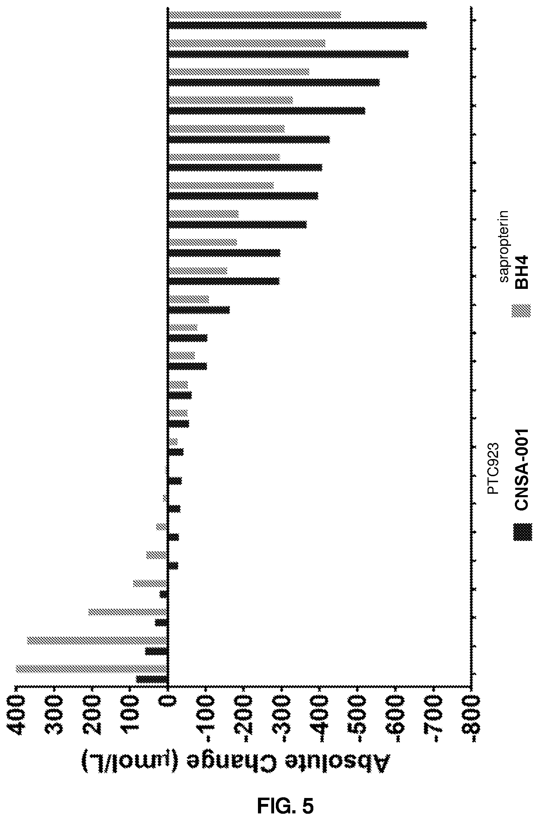

As shown in FIG. 5, PTC923 resulted in superior reduction in blood phenylalanine concentration compared to sapropterin dihydrochloride in in the study.

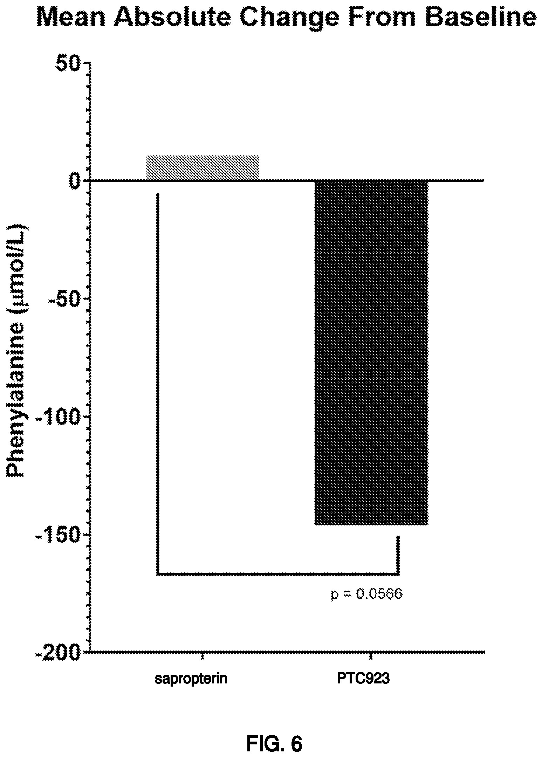

Least squares mean changes (SE; p value) in blood Phe in the 11 patients with classical PKU were -150.8 (63.1; p=0.0287) µmol/L for PTC923 60 mg/kg , -71.5 (61.8; p=0.2629) µmol/L for PTC923 20 mg/kg and -2.8 (62.0; p=0.9640) µmol/L for sapropterin dihydrochloride (Table 31). The comparison between PTC923 60 mg/kg vs. sapropterin dihydrochloride approached statistical significance (the LSM difference was -148.0 [SE 63.0; p=0.0566] µmol/L) (FIG. 6).

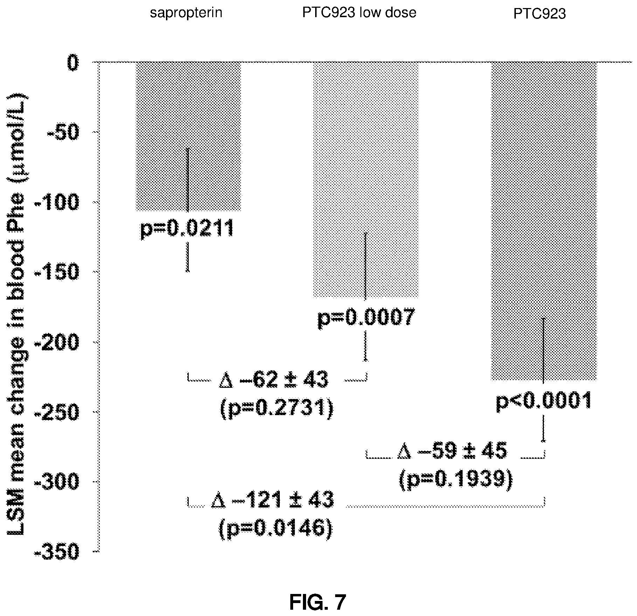

A sensitivity analysis was conducted that excluded treatment periods for patients with baseline blood Phe <300 µmol/L. Least squares mean changes (SE; p value) from baseline in blood Phe in the sensitivity analysis population were -226.9 (44.2; p<0.0001) µmol/L for PTC923 60 mg/kg, -167.8 (45.2; p=0.0007) µmol/L for PTC923 20 mg/kg, and -105.5 (43.7; p=0.0211) µmol/L for sapropterin dihydrochloride 20 mg/kg. The mean blood Phe reduction was significantly larger for PTC923 60 mg/kg vs. sapropterin dihydrochloride (the LSM difference was -121.4 [SE 42.9] µmol/L, p=0.0146) (FIG. 7).

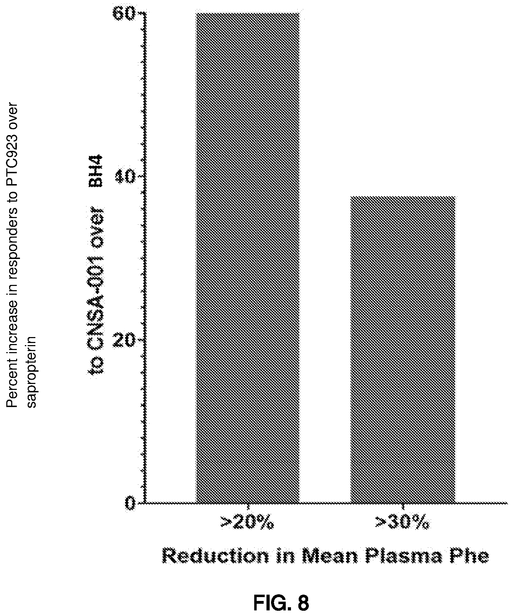

A cofactor responder analysis was also conducted in 19 patients with baseline Phe ≥300 µmol/L in all their treatment periods. Twelve patients (in any treatment group) out of 19 (63%) showed blood Phe reduction of ≥20%. Least squares mean changes (SE; p value) for this group of 12 responders from baseline in blood Phe were: -322.2 (60.0; p<0.0001) µmol/L for PTC923 60 mg/kg; - 234.8 (61.2; p=0.0011) µmol/L for PTC923 20 mg/kg; and -139.70 (58.6; p=0.0277) µmol/L for sapropterin dihydrochloride. The mean change in blood Phe reduction was significantly larger for PTC923 60 mg/kg vs. sapropterin dihydrochloride (the LSM difference was -182.5 [SE 62.0] µmol/L, p=0.0158) (FIG. 8). The number of patients treated with PTC923 showing blood Phe reduction of ≥20% was 50% higher than the number of patients treated with sapropterin. The absolute mean percentage change and the absolute Phe reduction in patients that were treated with PTC923 60mg/kg and showed at least 30% reduction in Phe from baseline was -72.5% and -485.3 µmol/L, respectively..

Similar numbers of patients (PTC923 60 mg/kg/PTC923 20 mg/kg/sapropterin dihydrochloride) reported any adverse event (AE; 29%/25%/21%), severe AE (0%/4%/0%), or treatment-related AE (13%/0%/0%). Headache was the most common all-cause AE (17%/4%/4%) and was transient in all cases.

No other individual AE occurred in ≥1 patient. Most AEs were mild in severity (19/22, 86% of all AEs). Two patients reported a moderate-severity AE (conjunctivitis, dysmenorrhea) and one patient reported a severe AE (gastrointestinal reflux disease); none of these three moderate to severe AEs were related to treatment. There were no SAEs and no patient discontinued due to AE. Changes in vital signs and ECG parameters were generally small and comparable across treatment groups.

The main objective of the study was to assess the efficacy of PTC923 measured as the mean blood Phe change from baseline. The efficacy population consisted of all patients who were randomized and had blood Phe measurements before treatment and at days 3, 5 and 7 of treatment from at least one treatment period. The safety population consisted of all patients who were randomized and who received any dose of study treatment. The sample size calculation was based on an all-comers, placebo-controlled regulatory study of sapropterin with an observed mean decrease in blood Phe of 99 µmol/L and standard deviation of 220 µmol/L, and assumed a true mean decrease in blood Phe of 300 µmol/L with 80% power utilizing a 2-sided α of 0.05.

Response to PTC923 was evaluated by measuring the mean change from baseline in blood Phe (calculated as the mean of measurements at days 3, 5 and 7 of each treatment period). Day 1 pre-dose blood Phe concentrations for each treatment period served as the individual patient specific baseline for that period.

The change from baseline in blood Phe of each treatment period was modelled using a linear mixed model for repeated measures (MMRM). The efficacy model included fixed effects for baseline for each treatment period, treatment group, sequence, period, and a random subject effect within each sequence, with a first order autoregressive AR (1) covariance structure. No unplanned covariates were included.

Pairwise comparisons in changes from baseline in Phe were performed between each PTC923 treatment and sapropterin treatment by calculating the least squares means with Dunnet's method of adjustment for multiple comparisons. The estimated change in blood Phe levels, their standard errors (SE) and associated p-values were calculated for each pairwise comparison. Dunnet's method of adjustment was used to account for the multiple comparisons of the test groups against the control group. This adjustment ensured that the overall Type I error rate under the null hypothesis was maintained despite multiple test groups being compared to the single control group.

The overall mean changes in blood Phe for each treatment were calculated and the standard deviations (SD) were determined.

A prespecified sensitivity analysis excluded treatment periods where baseline Phe was < 300 µmol/L and a responder analysis looked at patients with baselines ≥300 µmol/L who responded to sapropterin and/or to PTC923 treatment by ≥20% reduction.

Data were analysed using SAS v. 9.4.

Summary: Surprisingly, PTC923 resulted in statistically significant superiority in reduction of blood phenylalanine concentration over sapropterin dihydrochloride even though the study included only 24 subjects. Further, about 45% of classical PKU subjects responded to PTC923 with a decrease in phenylalanine levels of at least 20%. This is surprising because, historically, only about 10% of classical PKU subjects respond to sapropterin dihydrochloride treatment. PTC923 also had a surprisingly high overall level of responders compared to sapropterin dihydrochloride. 60% more subjects responded to PTC923 than to sapropterin dihydrochloride and 3-4 times the number of subjects were normalized, i.e., had a day 7 blood phenylalanine concentration of less than 120 micromole per liter, compared to treatment with sapropterin dihydrochloride. In fact, subjects on PTC923 were 13.45 times more like to reach normalized blood phenylalanine concentrations, i.e., less than 120 micromole per liter, compared to subjects on sapropterin dihydrochloride. In particular, 46% of non-classical subjects treated with PTC923 were normalized compared to 15.4% by sapropterin dihydrochloride. Subjects treated with PTC923 also reported improvement in sleep quality and better focus.

## Claims

1. Sepiapterin, or a pharmaceutically acceptable salt thereof, for use in a method of treating phenylketonuria in a subject having a blood phenylalanine concentration greater than 120 micromole per liter, optionally wherein the subject has a blood phenylalanine concentration greater than 600 micromole per liter;
wherein:
the subject failed to respond to treatment with sapropterin, or a pharmaceutically acceptable salt thereof, wherein the blood plasma phenylalanine concentration of the subject decreased less than 20% upon administration of sapropterin, or a pharmaceutically acceptable salt thereof; and
the method comprises administering to the subject an effective amount of the sepiapterin or pharmaceutically acceptable salt thereof.

2. The sepiapterin or salt for use according to claim 1, wherein:
the subject is on a phenylalanine-restricted diet.

3. The sepiapterin or salt for use according to claim 1 or 2, wherein:
the subject failed to respond to treatment with pegvaliase-pqpz;
optionally wherein the subject discontinued treatment with pegvaliase-pqpz due to an adverse reaction and/or tolerability.

4. The sepiapterin or salt for use according to any one of claims 1-3, wherein:
(a) the administering reduces the blood phenylalanine concentration of the subject to less than 120 micromole per liter;
(b) the administering reduces the blood phenylalanine concentration of the subject at least 35% compared to the blood phenylalanine concentration prior to administration of sepiapterin, or a pharmaceutically acceptable salt thereof;
(c) the administering reduces the blood phenylalanine concentration of the subject to less than 360 micromole per liter with a phenylalanine consumption of at least about 1500 mg/day; and/or
(d) the administering produces a BH4 concentration of at least 50 ng/mL in the plasma of the subject within 10 hours of administration of sepiapterin, or a pharmaceutically acceptable salt thereof.

5. The sepiapterin or salt for use according to any one of claims 1-4, wherein:
the effective amount of sepiapterin, or pharmaceutically acceptable salt thereof, is about 20 mg/kg to about 60 mg/kg per dose;
optionally wherein:
(a) the effective amount of sepiapterin, or pharmaceutically acceptable salt thereof, is about 20 mg/kg per dose;
(b) the effective amount of sepiapterin, or pharmaceutically acceptable salt thereof, is about 40 mg/kg per dose; or
(c) the effective amount of sepiapterin, or pharmaceutically acceptable salt thereof, is about 60 mg/kg per dose.

6. The sepiapterin or salt for use according to claim 5, wherein:
the risk of adverse events is reduced compared to a subject administered at least 10 mg/kg sapropterin, or a pharmaceutically acceptable salt thereof.

7. The sepiapterin or salt for use according to any one of claims 1-6, wherein:
the effective amount of sepiapterin, or pharmaceutically acceptable salt thereof, is administered once daily.

8. The sepiapterin or salt for use according to any one of claims 1-6, wherein:
the effective amount of sepiapterin, or pharmaceutically acceptable salt thereof, is administered twice daily;
preferably wherein the effective amount of sepiapterin, or pharmaceutically acceptable salt thereof, is administered in two equal doses.

9. The sepiapterin or salt for use according to any one of claims 1-8, wherein:
the effective amount of sepiapterin, or a pharmaceutically acceptable salt thereof, is administered with food;
optionally wherein:
(a) administration to the subject occurs less than 30 minutes prior to consuming food or after consuming food;
preferably wherein the administration to the subject is substantially at the same time as food;
(b) the food is high protein and/or high fat food; and/or
(c) the food is high calorie food.

10. The sepiapterin or salt for use according to any one of claims 1-9, wherein:
the administering produces an increase in neurocognitive function of the subject.

11. The sepiapterin or salt for use according to any one of claims 1-10, wherein:
the subject is a child;
optionally wherein the child is less than seven years old.

12. The sepiapterin or salt for use according to any one of claims 1-11, wherein:
the subject is more than seven years old.

13. The sepiapterin or salt for use according to any one of claims 1-12, wherein:
the subject has been diagnosed with sepiapterin-responsive phenylketonuria.

14. The sepiapterin or salt for use according to any one of claims 1-13, wherein:
the sepiapterin or pharmaceutically acceptable salt thereof is formulated as an oral powder for suspension;
optionally wherein the sepiapterin or pharmaceutically acceptable salt thereof is administered as a suspension in a flavored suspending vehicle;
optionally wherein the sepiapterin or pharmaceutically acceptable salt thereof is administered in a pharmaceutical composition with no antioxidant.

15. The sepiapterin or salt for use according to any one of claims 1-14, wherein:
the subject has a blood phenylalanine concentration greater than 1200 micromole per liter.

## Patentansprüche

1. Sepiapterin oder ein pharmazeutisch annehmbares Salz davon zur Verwendung in einem Verfahren zur Behandlung von Phenylketonurie in einem Individuum, das eine Phenylalanin-Konzentration im Blut von mehr als 120 µmol/I aufweist, wobei das Individuum gegebenenfalls eine Phenylalanin-Konzentration im Blut von mehr als 600 µmol/I aufweist; wobei:
das Individuum nicht auf eine Behandlung mit Sapropterin oder einem pharmazeutisch annehmbaren Salz davon angesprochen hat, wobei die Phenylalanin-Konzentration im Blutplasma des Individuums nach Verabreichung von Sapropterin oder einem pharmazeutisch annehmbaren Salz davon um weniger als 20 % abgenommen hat; und
das Verfahren das Verabreichen einer wirksamen Menge des Sepiapterins oder des pharmazeutisch annehmbaren Salzes davon umfasst.

2. Sepiapterin oder Salz davon zur Verwendung nach Anspruch 1, wobei:
das Individuum eine Phenylalanin-restriktive Ernährung einhält.

3. Sepiapterin oder Salz zur Verwendung nach Anspruch 1 oder 2, wobei:
das Individuum nicht auf eine Behandlung mit Pegvaliase-pqpz angesprochen hat;
wobei gegebenenfalls das Individuum die Behandlung mit Pegvaliase-pqpz aufgrund einer Nebenwirkung und/oder aus Verträglichkeitsgründen abgebrochen hat.

4. Sepiapterin oder Salz zur Verwendung nach einem der Ansprüche 1 bis 3, wobei:
(a) die Verabreichung die Phenylalanin-Konzentration im Blut des Individuums auf weniger als 120 µmol/l reduziert;
(b) die Verabreichung die Phenylalanin-Konzentration im Blut des Individuums im Vergleich zu der Phenylalanin-Konzentration im Blut vor der Verabreichung von Sepiapterin oder eines pharmazeutisch annehmbaren Salzes davon um zumindest 35 % reduziert;
(c) die Verabreichung die Phenylalanin-Konzentration im Blut des Individuums bei einer Phenylalanin-Aufnahme von zumindest 1.500 mg/Tag auf weniger als 360 µmol/I reduziert; und/oder
(d) die Verabreichung eine BH4-Konzentration von zumindest 50 ng/ml im Plasma des Individuums innerhalb von 10 h nach der Verabreichung von Sepiapterin oder einem pharmazeutisch annehmbaren Salz davon erzeugt.

5. Sepiapterin oder Salz zur Verwendung nach einem der Ansprüche 1 bis 4, wobei:
die wirksame Menge von Sepiapterin oder einem pharmazeutisch annehmbaren Salz davon etwa 20 mg/kg bis etwa 60 mg/kg pro Dosis beträgt;
wobei gegebenenfalls:
(a) die wirksame Menge an Sepiapterin oder einem pharmazeutisch annehmbaren Salz davon etwa 20 mg/kg pro Dosis beträgt;
(b) die wirksame Menge an Sepiapterin oder einem pharmazeutisch annehmbaren Salz davon etwa 40 mg/kg pro Dosis beträgt; oder
(c) die wirksame Menge an Sepiapterin oder einem pharmazeutisch annehmbaren Salz davon etwa 60 mg/kg pro Dosis beträgt.

6. Sepiapterin oder Salz zur Verwendung nach Anspruch 5, wobei:
das Risiko von Nebenwirkungen im Vergleich mit einem Individuum, dem zumindest 10 mg/kg von Sapropterin oder einem pharmazeutisch annehmbaren Salz davon verabreicht wurden, reduziert ist.

7. Sepiapterin oder Salz zur Verwendung nach einem der Ansprüche 1 bis 6, wobei:
die wirksame Menge an Sepiapterin oder einem pharmazeutisch annehmbaren Salz davon einmal täglich verabreicht wird.

8. Sepiapterin oder Salz zur Verwendung nach einem der Ansprüche 1 bis 6, wobei:
die wirksame Menge an Sepiapterin oder einem pharmazeutisch annehmbaren Salz davon zweimal täglich verabreicht wird;
wobei vorzugsweise die wirksame Menge an Sepiapterin oder einem pharmazeutisch annehmbaren Salz davon in zwei gleichen Dosen verabreicht wird.

9. Sepiapterin oder Salz zur Verwendung nach einem der Ansprüche 1 bis 8, wobei:
die wirksame Menge an Sepiapterin oder einem pharmazeutisch annehmbaren Salz davon zusammen mit Nahrung verabreicht wird;
wobei gegebenenfalls:
(a) die Verabreichung an das Individuum weniger als 30 min vor Einnahme der Nahrung oder nach Einnahme der Nahrung erfolgt;
wobei vorzugsweise die Verabreichung an das Individuum im Wesentlichen zur gleichen Zeit wie die Einnahme der Nahrung erfolgt;
(b) die Nahrung eine Nahrung mit hohem Proteingehalt und/oder hohem Fettgehalt ist; und/oder
(c) die Nahrung eine hochkalorische Nahrung ist.

10. Sepiapterin oder Salz zur Verwendung nach einem der Ansprüche 1 bis 9, wobei:
die Verabreichung einen Anstieg der neurokognitiven Funktion des Individuums erzeugt.

11. Sepiapterin oder Salz zur Verwendung nach einem der Ansprüche 1 bis 10, wobei:
das Individuum ein Kind ist;
wobei gegebenenfalls das Kind jünger als sieben Jahre ist.

12. Sepiapterin oder Salz zur Verwendung nach einem der Ansprüche 1 bis 11, wobei:
das Individuum älter als sieben Jahre ist.

13. Sepiapterin oder Salz zur Verwendung nach einem der Ansprüche 1 bis 12, wobei:
bei dem Individuum eine auf Sepiapterin ansprechende Phenylketonurie diagnostiziert wurde.

14. Sepiapterin oder Salz zur Verwendung nach einem der Ansprüche 1 bis 13, wobei:
das Sepiapterin oder das pharmazeutisch annehmbare Salz davon als orales Pulver zur Suspension formuliert ist;
wobei gegebenenfalls das Sepiapterin oder das pharmazeutisch annehmbare Salz davon als Suspension in einem aromatisierten Suspendier-Vehikel verabreicht wird;
wobei gegebenenfalls das Sepiapterin oder das pharmazeutisch annehmbare Salz davon in einer pharmazeutischen Zusammensetzung ohne Antioxidans verabreicht wird.

15. Sepiapterin oder Salz zur Verwendung nach einem der Ansprüche 1 bis 14, wobei:
das Individuum eine Phenylalanin-Konzentration im Blut von mehr als 1.200 µm/I aufweist.

## Revendications

1. Sépiaptérine, ou un sel pharmaceutiquement acceptable de celle-ci, pour utilisation dans un procédé de traitement de la phénylcétonurie chez un sujet présentant une concentration de phénylalanine dans le sang supérieure à 120 micromoles par litre, facultativement dans laquelle le sujet présente une concentration de phénylalanine dans le sang supérieure à 600 micromoles par litre ; dans laquelle :
le sujet n'a pas répondu à un traitement par saproptérine, ou un sel pharmaceutiquement acceptable de celle-ci, dans laquelle la concentration de phénylalanine dans le plasma sanguin du sujet a diminué de moins de 20 % lors de l'administration de saproptérine, ou d'un sel pharmaceutiquement acceptable de celle-ci ; et
le procédé comprend une administration au sujet d'une quantité efficace de la sépiaptérine ou d'un sel pharmaceutiquement acceptable de celle-ci.

2. Sépiaptérine, ou sel, pour utilisation selon la revendication 1, dans laquelle :
le sujet suit un régime à restriction de phénylalanine.

3. Sépiaptérine, ou sel, pour utilisation selon la revendication 1 ou 2, dans laquelle :
le sujet n'a pas répondu à un traitement par pegvaliase-pqpz ;
facultativement dans laquelle le sujet a interrompu un traitement par pegvaliase-pqpz en raison d'un effet et/ou d'une tolérance indésirable.

4. Composition, ou sel, pour utilisation selon l'une quelconque des revendications 1 à 3, dans laquelle :
(a) l'administration réduit la concentration de phénylalanine dans le sang du sujet à moins de 120 micromoles par litre ;
(b) l'administration réduit la concentration de phénylalanine dans le sang du sujet d'au moins 35 % par comparaison à la concentration de phénylalanine dans le sang avant l'administration de sépiaptérine, ou d'un sel pharmaceutiquement acceptable de celle-ci ;
(c) l'administration réduit la concentration de phénylalanine dans le sang du sujet à moins de 360 micromoles par litre avec une consommation de phénylalanine d'au moins environ 1 500 mg/jour ; et/ou
(d) l'administration produit une concentration de BH4 d'au moins 50 ng/ml dans le plasma du sujet dans les 10 heures suivant l'administration de sépiaptérine, ou d'un sel pharmaceutiquement acceptable de celle-ci.

5. Composition, ou sel, pour utilisation selon l'une quelconque des revendications 1 à 4, dans laquelle :
la quantité efficace de sépiaptérine, ou d'un sel pharmaceutiquement acceptable de celle-ci, est d'environ 20 mg/kg à environ 60 mg/kg par dose ;
facultativement dans laquelle :
(a) la quantité efficace de sépiaptérine, ou d'un sel pharmaceutiquement acceptable de celle-ci, est d'environ 20 mg/kg par dose ;
(b) la quantité efficace de sépiaptérine, ou d'un sel pharmaceutiquement acceptable de celle-ci, est d'environ 40 mg/kg par dose ; ou
(c) la quantité efficace de sépiaptérine, ou d'un sel pharmaceutiquement acceptable de celle-ci, est d'environ 60 mg/kg par dose.

6. Composition pour utilisation selon la revendication 5, dans laquelle :
le risque d'événements indésirables est réduit par comparaison à un sujet s'étant vu administrer au moins 10 mg/kg de saproptérine, ou d'un sel pharmaceutiquement acceptable de celle-ci.

7. Composition, ou sel, pour utilisation selon l'une quelconque des revendications 1 à 6, dans laquelle :
la quantité efficace de sépiaptérine, ou d'un sel pharmaceutiquement acceptable de celle-ci, est administrée une fois par jour.

8. Composition, ou sel, pour utilisation selon l'une quelconque des revendications 1 à 6, dans laquelle :
la quantité efficace de sépiaptérine, ou d'un sel pharmaceutiquement acceptable de celle-ci, est administrée deux fois par jour ;
de préférence dans laquelle la quantité efficace de sépiaptérine, ou d'un sel pharmaceutiquement acceptable de celle-ci, est administrée en deux doses égales.

9. Composition, ou sel, pour utilisation selon l'une quelconque des revendications 1 à 8, dans laquelle :
la quantité efficace de sépiaptérine, ou d'un sel pharmaceutiquement acceptable de celle-ci, est administrée avec de la nourriture ;
facultativement dans laquelle :
(a) l'administration au sujet a lieu moins de 30 minutes avant de consommer des aliments ou après avoir consommé des aliments ;
de préférence dans laquelle l'administration au sujet est sensiblement en même temps qu'un aliment ;
(b) l'aliment est un aliment riche en protéines et/ou en matières grasses ; et/ou
(c) l'aliment est un aliment riche en calories.

10. Composition, ou sel, pour utilisation selon l'une quelconque des revendications 1 à 9, dans laquelle :
l'administration produit une augmentation de la fonction neurocognitive du sujet.

11. Composition, ou sel, pour utilisation selon l'une quelconque des revendications 1 à 10, dans laquelle :
le sujet est un enfant ;
facultativement dans laquelle l'enfant est âgé de moins de sept ans.

12. Composition, ou sel, pour utilisation selon l'une quelconque des revendications 1 à 11, dans laquelle :
le sujet a plus de sept ans.

13. Composition, ou sel, pour utilisation selon l'une quelconque des revendications 1 à 12, dans laquelle :
le sujet a reçu un diagnostic de phénylcétonurie sensible à la sépiaptérine.

14. Composition, ou sel, pour utilisation selon l'une quelconque des revendications 1 à 13, dans laquelle :
la sépiaptérine, ou un sel pharmaceutiquement acceptable de celle-ci, est formulée sous forme de poudre orale pour suspension ;
facultativement dans laquelle la sépiaptérine, ou un sel pharmaceutiquement acceptable de celle-ci, est administrée sous forme de suspension dans un excipient de suspension aromatisé ;
facultativement dans laquelle la sépiaptérine, ou un sel pharmaceutiquement acceptable de celle-ci, est administrée dans une composition pharmaceutique sans antioxydant.

15. Composition, ou sel, pour utilisation selon l'une quelconque des revendications 1 à 14, dans laquelle :
le sujet présente une concentration de phénylalanine dans le sang supérieure à 1 200 micromoles par litre.
